# EUROPEAN PATENT SPECIFICATION

(11) **EP 1 753 354 B1**
(45) Date of publication and mention of the grant of the patent: **15.09.2010**
(21) Application number: 05752031.4
(22) Date of filing: 20.05.2005
(51) Int. Cl.: A61B 17/72

(54) **FRACTURE FIXATION AND SITE STABILIZATION SYSTEM**
FRAKTURFIXATIONS- UND SITUSSTABILISATIONSSYSTEM
SYSTÈME DE FIXATION ET DE STABILISATION DE SITE DE FRACTURE

(30) Priority: 21.05.2004 US 573561 P
(43) Date of publication of application: 21.02.2007
(73) Proprietor: Myers Surgical Solutions, LLC, MARIETTA, GA 30067-9123 (US)
(72) Inventor: MYERS, Thomas, H., Marietta, GA 30067-9123 (US); LORANG, Douglas, M., Ripon, CA 95366-9218 (US)
(74) Representative: Martin, Didier Roland Valéry
(86) International application number: PCT/US2005/017807
(87) International publication number: WO 2005/112804

(56) References cited:
- WO-A-01/28443
- WO-A-98/56301

## Description

### BACKGROUND

Technical Field: The following disclosure relates generally to the treatment of bone conditions in humans and other animals and, more particularly, to the fixation and stabilization of fracture sites, especially in long bones.

### Description of Related Art: Current systems and methods for the fixation of bone fractures of the appendicular skeleton involve external immobilization of the fracture with casts, splinting devices or external fixation frames, internal fixation with plates and screws, or indirect fixation of the fracture by insertion of an intramedullary device.

Some of these prior art devices currently use compression plates and screw devices to apply a compression force across the fracture site. However, for insertion of this type of device, it is typically necessary to make a large surgical incision over the outer cortex of the bone directly at the fracture site. Installing plates and screws usually requires the disturbance of the soft tissues overlying the fracture site, disturbance of the fracture hematoma, and stripping the periosteum of bone which compromises the blood supply to the fracture fragments. Moreover, the application of a compressive force alone is generally not sufficient to fix and stabilize a bone fracture, especially in long bones such as the human femur, tibia, and distal radius.

Another system for treating a fracture site includes intramedullary nailing, wherein one or more nails are inserted into the intramedullary canal of a fractured bone, usually through an incision located at either end of the bone, as described for example in U.S. Patent 4,457,301 issued to Walker in 1984. Intramedullary nailing offers some advantages over external casting and some other methods of fracture stabilization. The biomechanical advantages of nailing include load sharing along the central axis of the bone, torsional stabilization of the fracture proximal and distal to the fracture site, and the nail's resistance to compression and bending forces. Biological advantages include preservation of the soft tissue envelope at the fracture site, preservation of blood supply to the fracture site, and formation of abundant bone callous around the fracture due to micro-motion of the fragments. Also, surgical advantages include small incisions remote from the fracture through non-trumatized tissues, ease of insertion of the fixation device, and use of the device itself for fracture reduction.

There are, however, many disadvantages associated with intramedullary nailing. Both the reaming of the intramedullary canal and the placement of a nail without reaming compromise the intramedullary blood supply to the fracture. The act of instrumenting the canal itself has been shown to embolize fat and marrow contents into the vascular system, which can have adverse health effects. Insertion of the nail typically requires a direct line of sight down the canal. Acquiring a direct line of sight often requires incisions at either the proximal or distal end of the bone and violation of joints or tendon/ligament insertions in order to expose a starting point for entrance into the medullary canal. Exposing the starting point for nail insertion is a significant cause of post-operative complications that can eventually require removal of the implants or other surgical procedures. Moreover, the ends of long bones in children are also the growth center of the bones. Drilling or gouging through these epiphyseal plates may cause growth arrest and may lead to deformity or length discrepancy.

Placement of interlocking screws through the nail requires separate incisions, technical skill, and increased procedure time. Additionally, the stability of a bone implant construct is completely dependant on the size and strength of the interlocking screws. Another disadvantage of intramedullary nailing is the inability of the intramedullary device to "fit and fill" the medullary canal. The mismatch between the cross-sectional geometry of the bone and the nail places all the contact forces on the proximal and distal interlocking screws. The failure of small unreamed nails is likely due to tangential contact between the nail and the endosteal surface, putting the interlocking screws at a biomechanical disadvantage. Further, the biomechanical properties of these implants, often made of titanium or stainless steel, do not resemble those of the surrounding bone. Differences in the elastic modulus and isotropic features of the implant can lead to stress risers at the ends of the implant and an eventual failure in the form of re-fracture at these interfaces. It is also known from WO-01/28 443 a system for treating a fracture site as mentioned in the preamble of claim 1.

However, there exists a need in the art for a less invasive and more effective method of stabilizing a bone fracture site with minimal disruption of the fracture biology, reduced trauma to the intramedullary canal, better biomechanical properties, and smaller incisions. There is also a need in the art for stabilizing fracture sites in children without insulting the epiphyseal growth plates. There is a related need in the art for a method of efficiently delivering any of a variety of biological mediators directly to a fracture site to promote healing.

### SUMMARY OF THE INVENTION

The invention is defined in claim 1. Preferred embodiments are defined in dependent claims 2-49.

The above and other needs are met by the present invention.

The present invention provides a heath for use in a system for treating a fracture site in a bone having an intramedullary canal. The sheath may be characterized by a size and shape suitable for insertion into the canal, a length sufficient to span the fracture site, an elongate tubular structure sized and shaped to receive a structural frame extending through the canal.

The structural frame and sheath, together, as described above, are provided for use in a system for treating a fracture site in a bone having an intramedullary canal. In another aspect, the present invention also provides a hardenable surgical fluid, cooperative with the structural frame, to provide additional support across the fracture site.

These and other objects are accomplished by the present invention and will become apparent from the following detailed description of a preferred embodiment in conjunction with the accompanying drawings in which like numerals designate like elements.

### BRIEF DESCRIPTION OF THE DRAWING

The invention may be more readily understood by reference to the following description, taken with the accompanying drawing figures, in which:
Figure 1 is an illustration of a fracture site and a stabilization system.
Figure 2 is a closer illustration of a fracture site and a stabilization system.
Figure 3 is a cross-sectional illustration of a bone and intramedullary canal at a fracture site.
Figure 4 is a perspective illustration of a sheath surrounding a structural frame.
Figure 5 is a perspective illustration of a structural frame positioned near a fracture site and covered by a retainer.
Figure 6 is a perspective illustration of the structural frame depicted in Figure 5, showing the frame expanding after proximal movement of the retainer.
Figure 7 is a perspective illustration of a modified structural frame positioned near a fracture site and expandable by an internal force.
Figure 8 is a perspective illustration of a structural frame with an internal sheath shown in cutaway.
Figure 9 is a perspective side view of the structural frame depicted in Figure 8, showing the frame expanding after proximal movement of a retaining sleeve.
Figure 10 is a perspective view of a modified construction of the structural frame.
Figure 11A is an illustration of a periprosthetic fracture site and a prosthesis.

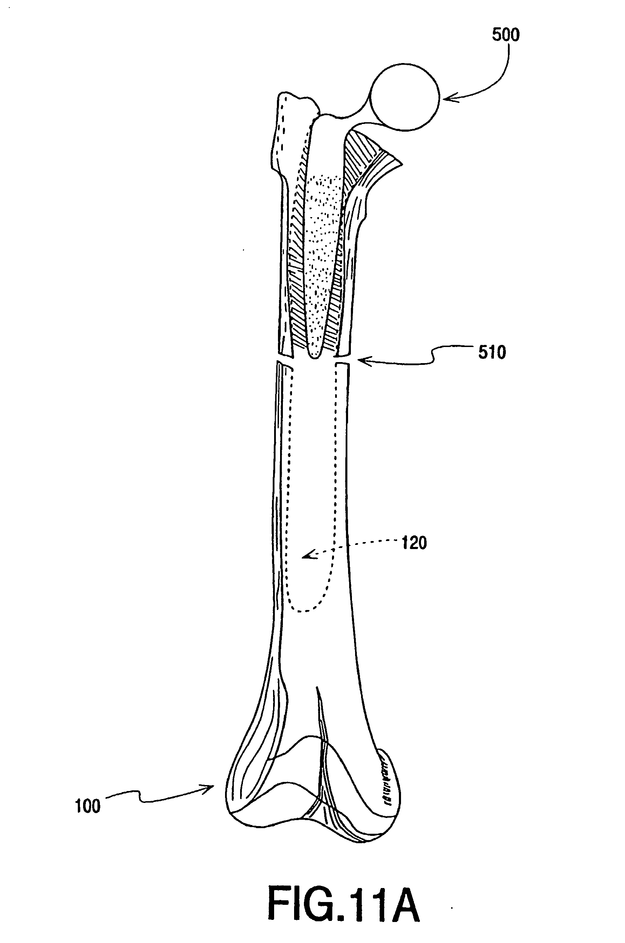

Figure 11A is an illustration of a periprosthetic fracture site and a stabilization system.

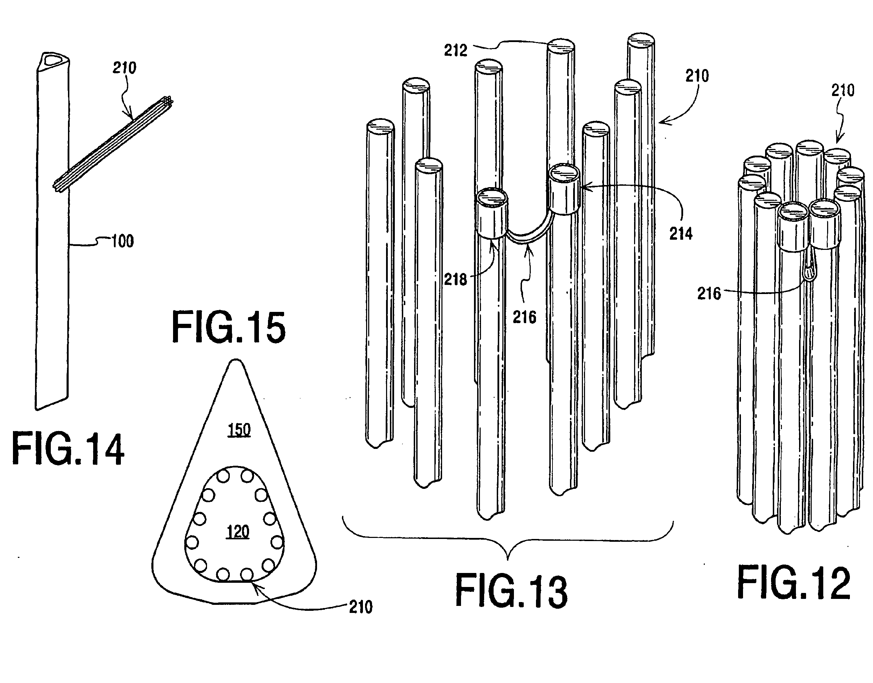

Figure 12 is an enlarged perspective view of another embodiment of the structural frame.
Figure 13 is an enlarged perspective view of the structural frame depicted in Figure 12, shown in an expanded condition.
Figure 14 is a perspective view of the structural frame depicted in Figure 12, showing insertion into a bone.
Figure 15 is a cross-sectional view of a bone and its intramedullary canal, showing the structural frame depicted in Figures 12-14 which has been inserted and expanded.

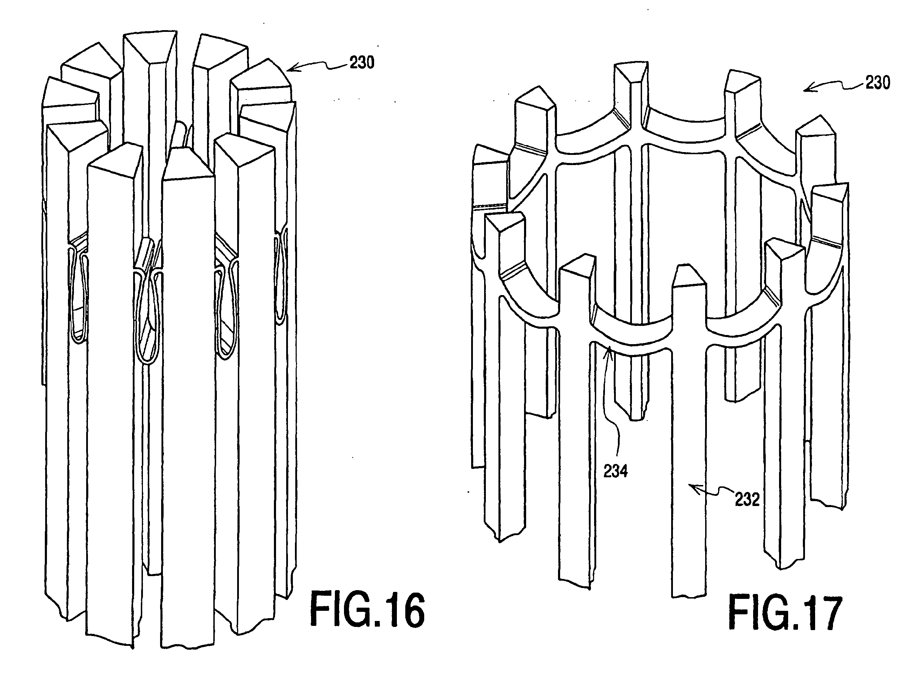

Figure 16 is a perspective view of another structural frame.
Figure 17 is a perspective view of the structural frame depicted in Figure 16, except the frame is shown in an expanded condition.

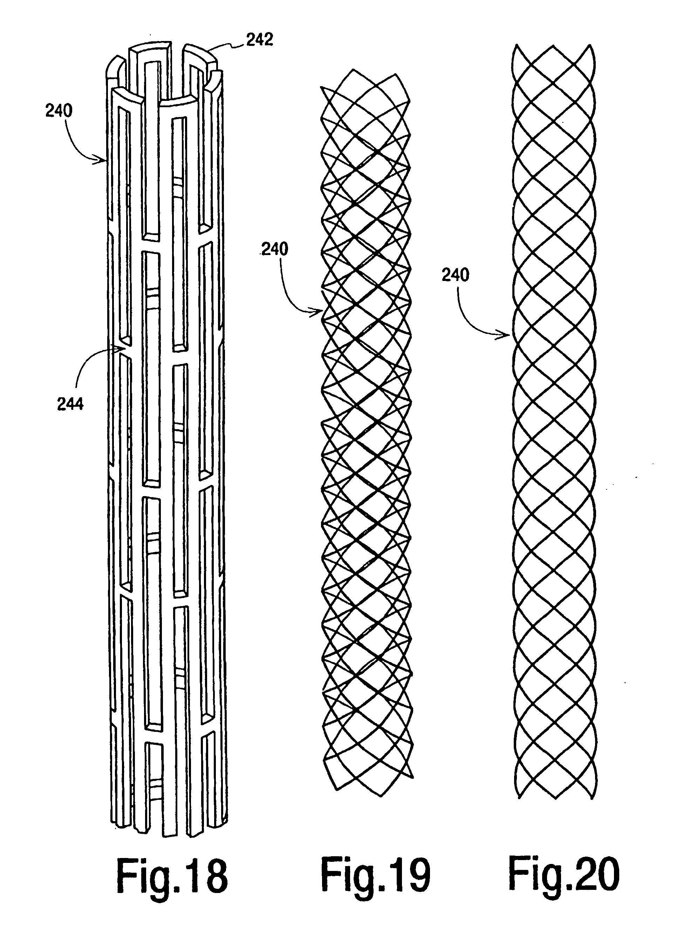

Figure 18 is a perspective view of an additional embodiment for the structural frame.
Figure 19 is a diagrammatic perspective view of the structural frame.
Figure 20 is a diagrammatic front view of the structural frame depicted in Figure 19.

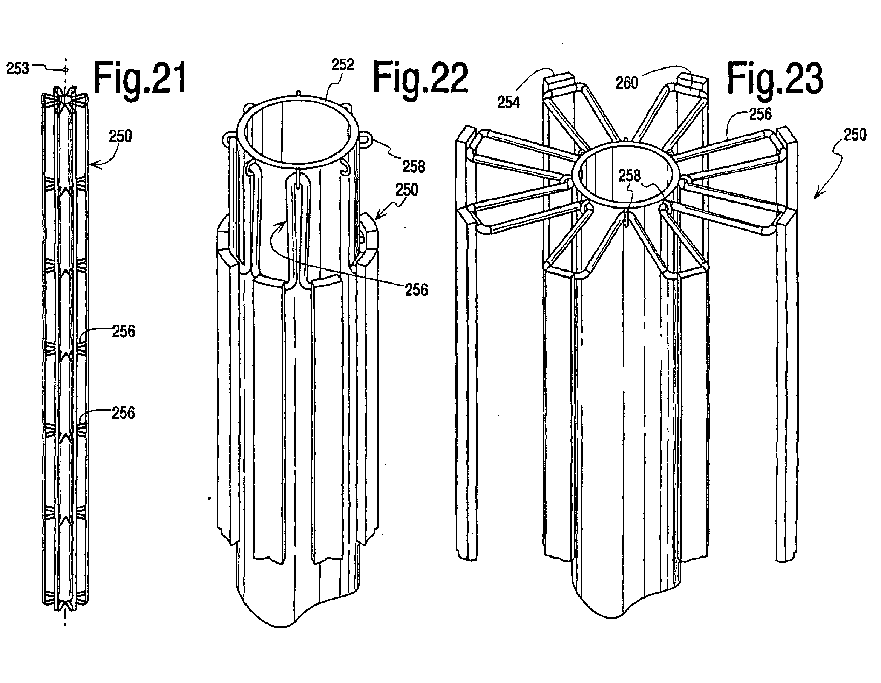

Figure 21 is an enlarged perspective view of yet another embodiment of the structural frame, shown in an unexpanded condition.
Figure 22 is an enlarged perspective view of the structural frame depicted in Figure 21, shown in an unexpanded condition and showing portions of the linking members removed for clarity.
Figure 23 is a perspective view of the structural frame depicted in Figures 21 and 22, shown in an expanded condition.

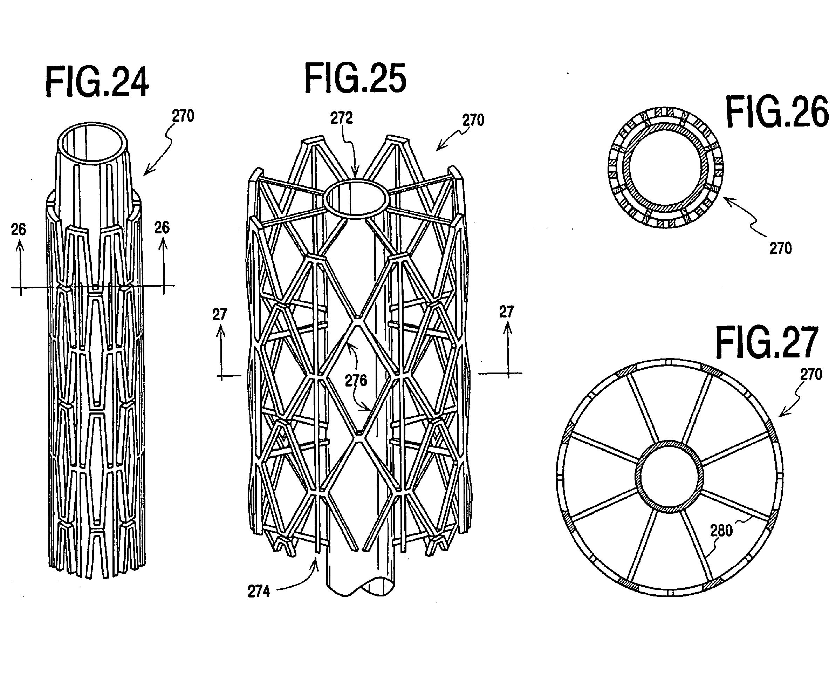

Figure 24 is a perspective view of yet a further embodiment of the structural frame, shown in an unexpanded condition.
Figure 25 is an enlarged perspective view of the structural frame depicted in Figure 24, shown in an expanded condition.
Figure 26 is a cross-sectional view of the structural frame depicted in Figure 24, in a unexpanded condition, taken along plane 26-26.
Figure 27 is a cross-sectional view of the structural frame depicted in Figure 24, in an expanded condition, taken along plane 27-27.

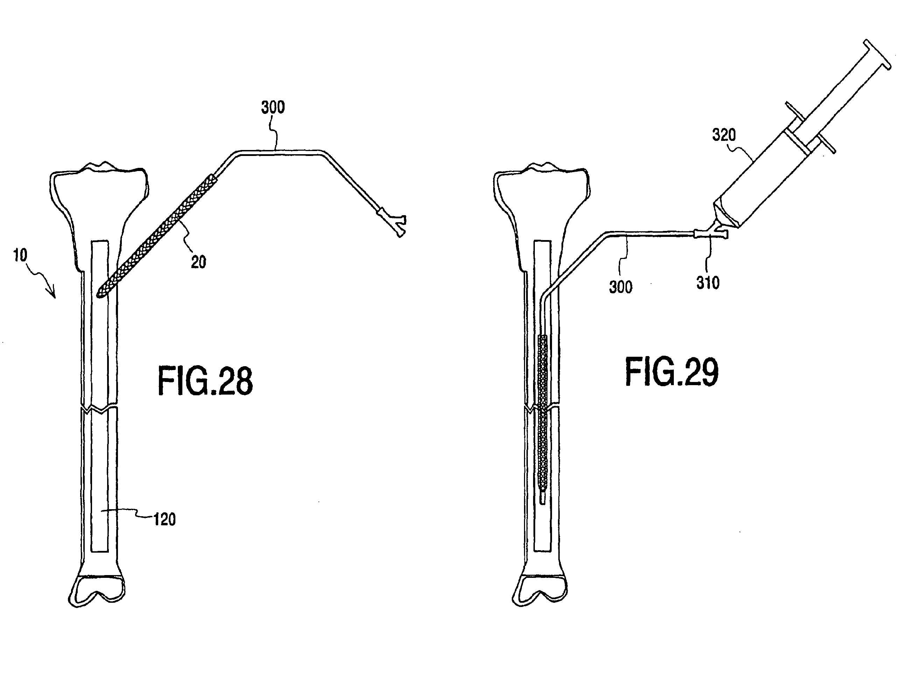

Figure 28 is a sectional view of a structural frame being inserted into a bone.
Figure 29 is a sectional view of the structural frame depicted in Figure 28, shown in an inserted and unexpanded condition.

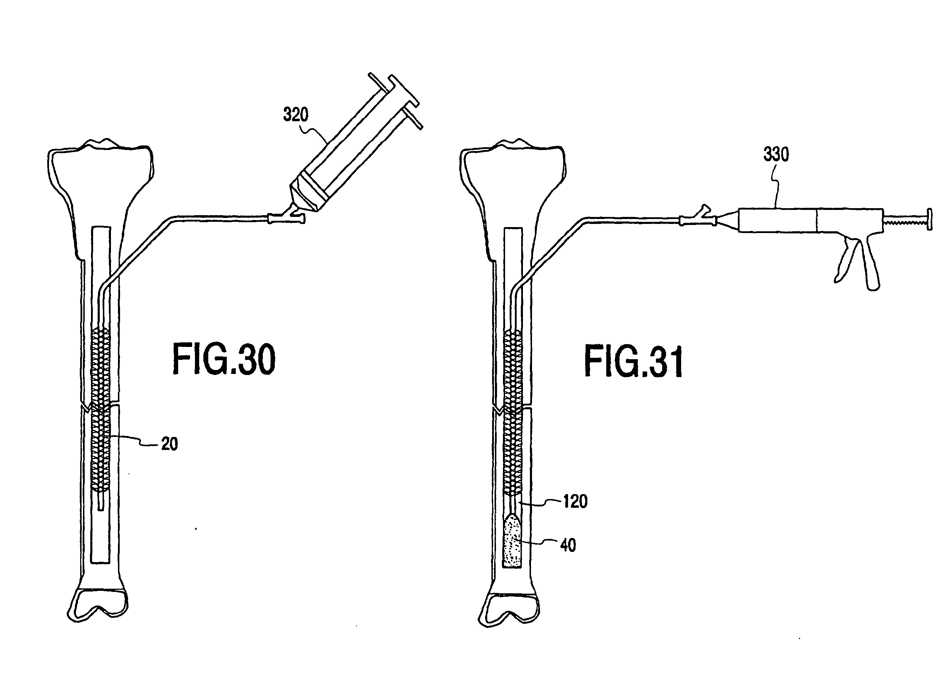

Figure 30 is a sectional view of the structural frame depicted in Figure 29, shown in an inserted and expanded condition.
Figure 31 is a sectional view similar to Figures 28-30, showing a retrograde insertion of a surgical fluid.

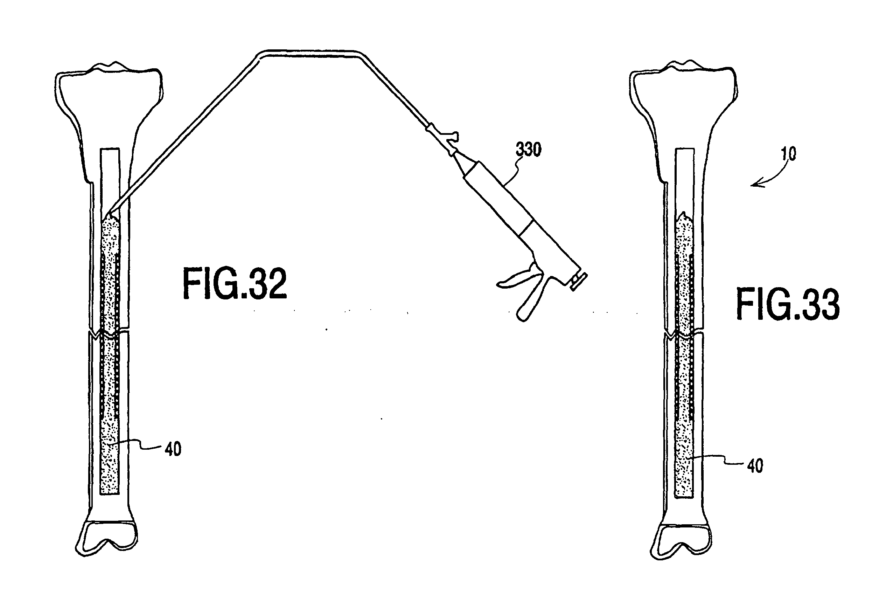

Figure 32 is a sectional view similar to Figures 28-31, showing the retrograde insertion of a surgical fluid.
Figure 33 is a sectional view of the structural frame depicted in Figures 28-32, as installed.

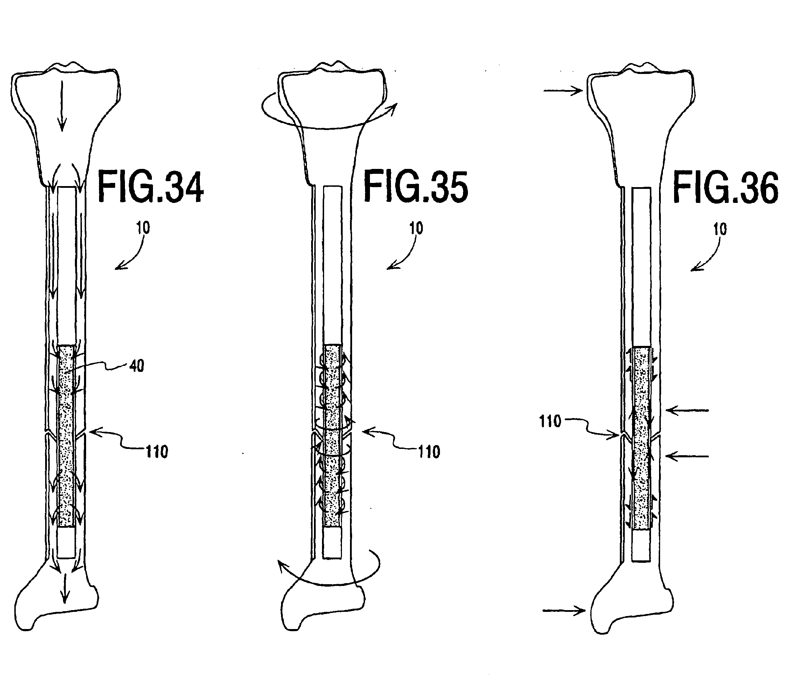

Figure 34 is a sectional view of a structural frame positioned across a fracture site, showing the transfer of forces when a compressive force (squeezing force) is applied to the bone.
Figure 35 is a sectional view of a structural frame positioned across a fracture site, showing the transfer of forces when a torsional force (twisting force) is applied to the bone.
Figure 36 is a sectional view of a structural frame positioned across a fracture site, showing the transfer of forces when a lateral force (bending force) is applied to the bone.

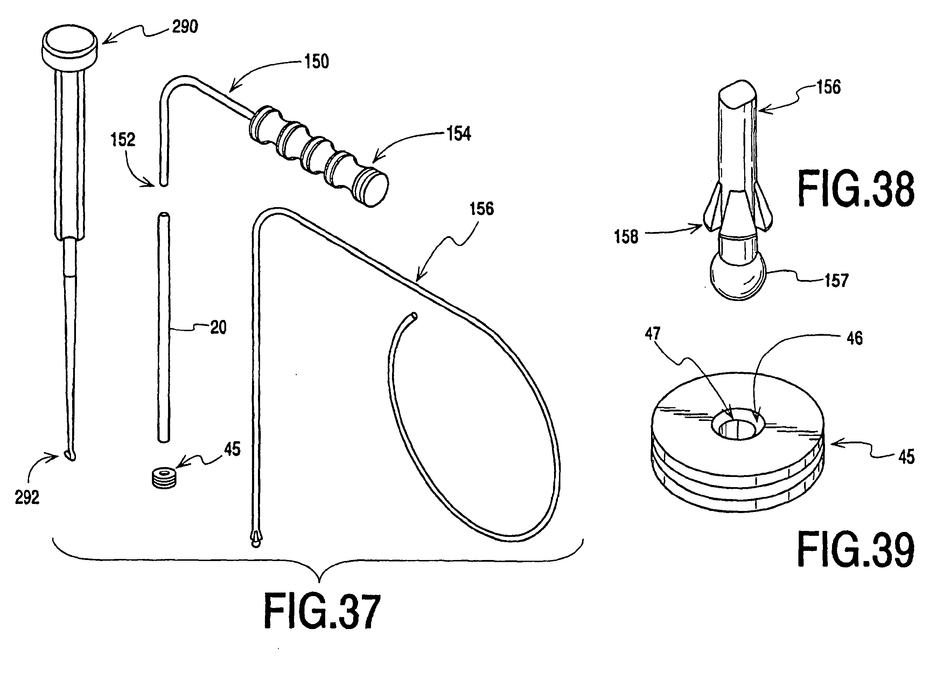

Figure 37 is a perspective view of the guidance tools for a stabilization system.
Figure 38 is an enlarged perspective view of a distal end of the guide wire depicted in Figure 37.
Figure 39 is an enlarged perspective view of the fluid restrictor depicted in Figure 37.

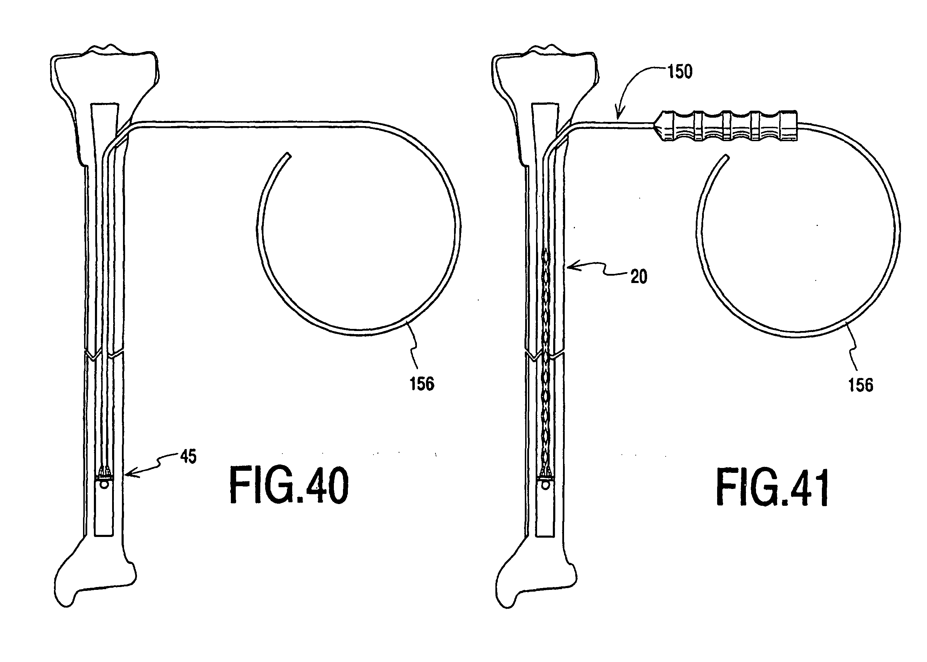

Figure 40 is a sectional view of a fracture site showing insertion of a guide wire and fluid restrictor.
Figure 41 is a sectional view of a fracture site showing insertion a structural frame.

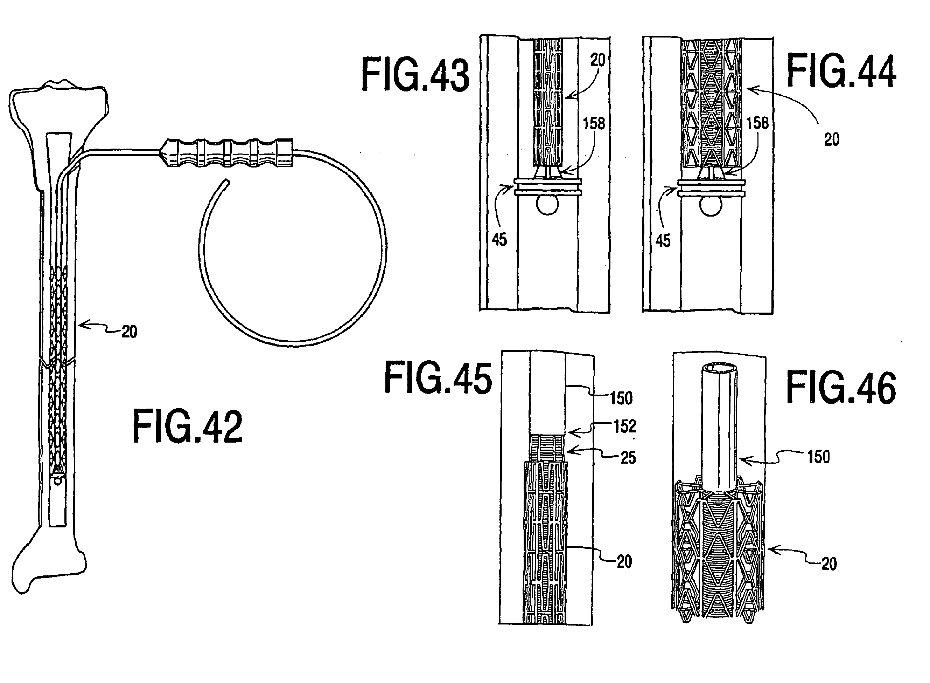

Figure 42 is a sectional view of a fracture site showing a structural frame in an expanded condition.
Figure 43 is an enlarged side elevation of the distal end of the structural frame, the guide wire, and the fluid restrictor.
Figure 44 is an enlarged side elevation similar to Figure 43 except the structural frame is shown in an expanded condition.
Figure 45 is an enlarged side elevation of the proximal end of the structural frame, in an unexpanded condition.
Figure 46 is an enlarged side elevation similar to Figure 45 except the structural frame is shown in an expanded condition.

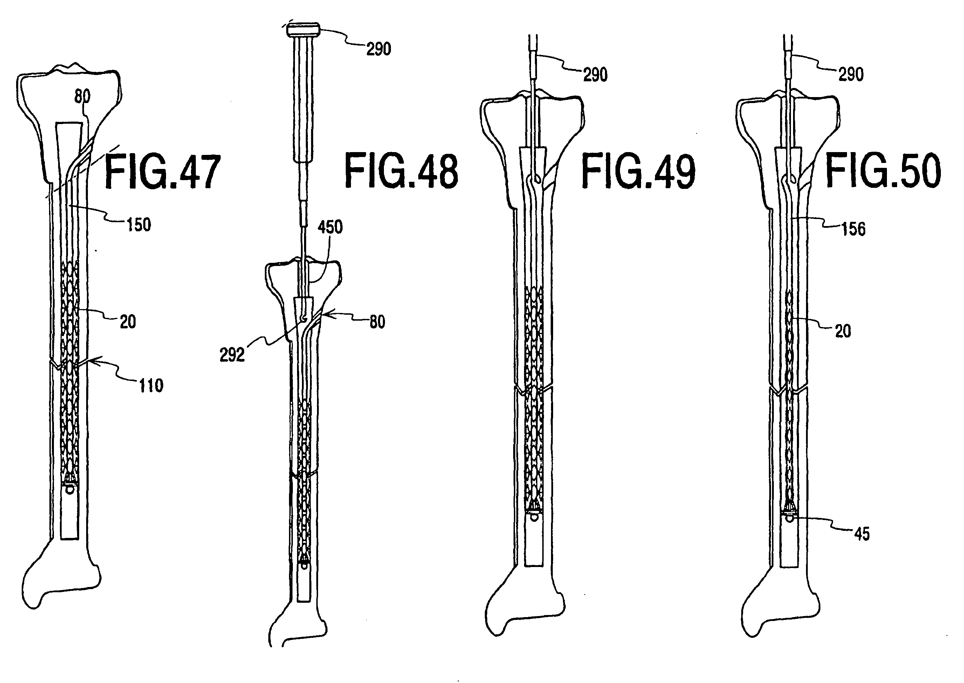

Figure 47 is a sectional view of the fracture site after placement of the structural frame and after the guide wire has been cut near the surface of the bone.
Figure 48 is a sectional view of the fracture site showing a retrieval tool.
Figure 49 is a sectional view of the fracture site showing a retrieval tool grasping a structural frame, in its expanded condition.
Figure 50 is a sectional view of the fracture site showing a retrieval tool grasping a structural frame, in its unexpanded condition, and showing removal of the stabilization system.

### DETAILED DESCRIPTION

### Introduction

Exemplary systems are now described with reference to the drawing figures, where like reference numerals are used to refer to like elements throughout the several views. In the following description, for purposes of explanation, numerous specific details are set forth in order to facilitate a thorough understanding of the systems. It may be evident, however, that the exemplars described may be practiced without these specific details. In other instances, common structures and devices are shown in block diagram form in order to simplify the description.

Although the systems will will be more specifically described in the context of the treatment of long bones such as the human femur or tibia, other human or animal bones, of course, may be treated in the same or similar fashion.

### Internal Reinforcement System

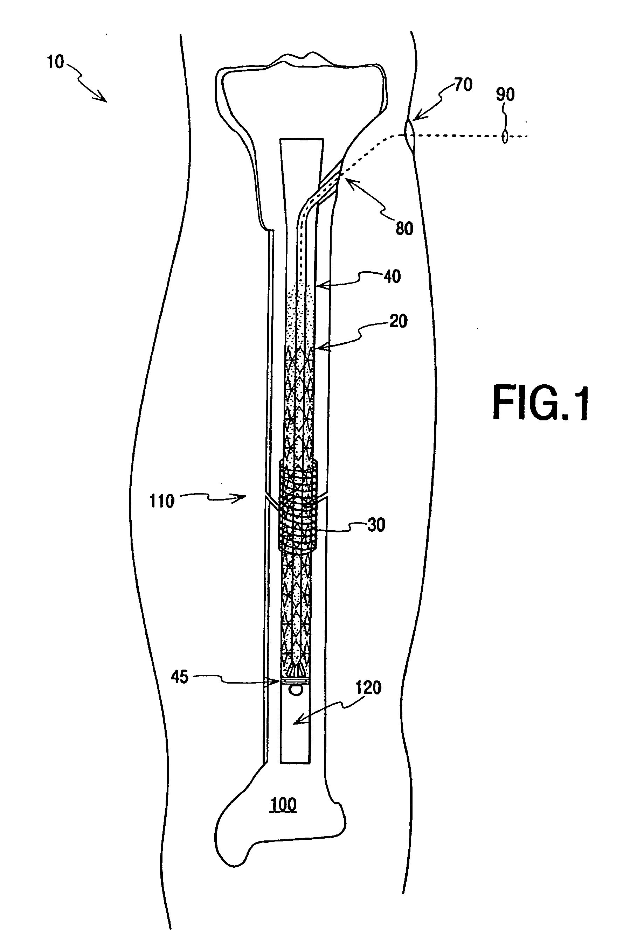

In one embodiment, as shown in Figure 1, the system 10 includes a structural frame 20 positioned within the intramedullary canal 120 of a bone 100, at least partially surrounded by a flexible sheath 30, and filled with a column of surgical fluid 40 such as polymer cement. As shown, the system 10 may be positioned to span a fracture site 110. The system 10 may be introduced into the intramedullary canal 120 through an incision 70 in the skin and an opening or breach 80 in the bone, along a path 90 which may proceed along an approximately centerline through the canal 120. Once in place, the cement or other surgical fluid 40 hardens and, together with the structural frame 20, provides fixation and stabilization of the fracture site. The surgical fluid 40 may be contained at least partially by a sheath 30, positioned near the fracture site 110, in order to prevent the fluid 40 from seeping into the fracture site 110.

As shown in Figure 1, the structural frame 20 may be configured for placement within the intramedullary cavity 120. The exemplary bone 100 shown in Figure 1 includes an intramedullary canal 120 that passes through the interior of the bone near its central axis. In adults, the canal 120 in a long bone, such as the tibia in the leg, may be generally hollow. Marrow production in adult long bones generally ceases over time, so the introduction of intramedullary devices generally does not compromise marrow production. Long bones are hard, dense bones that provide strength, structure, and mobility, regardless of their size or length. There are bones in the fingers, for example, that may be classified as long bones because of their shape and function. In general, long bones contain yellow bone marrow and red bone marrow, which produces red blood cells.

In one embodiment, one or more elements of the system 10 of the present invention may contain antibiotics, pharmaceuticals, or other compounds that prevent infection, promote healing, reduce pain, or otherwise improve the condition of the fracture site 110. Such pharmaceuticals or compounds may be designed to elute from the solid construct of the system 10 over time in order to provide therapeutic doses local to the fracture site 110 at desired times during the stabilization and healing process. For example, the system 10 of the present invention may comprise one or more delivery drugs or agents which facilitate healing, such as for example, as part of the material of the apparatus, or a portion thereof, so as to provide time-release delivery of such drug or agent when the system elements are positioned to span the fracture site 110. Some of these aspects will be described in further detail.

In general, the system 10 of the present invention may be used to provide fixation and stabilization of a fracture site 110 after other techniques have been performed to reduce, compress, distract, align, or otherwise manipulate the opposing fracture ends.

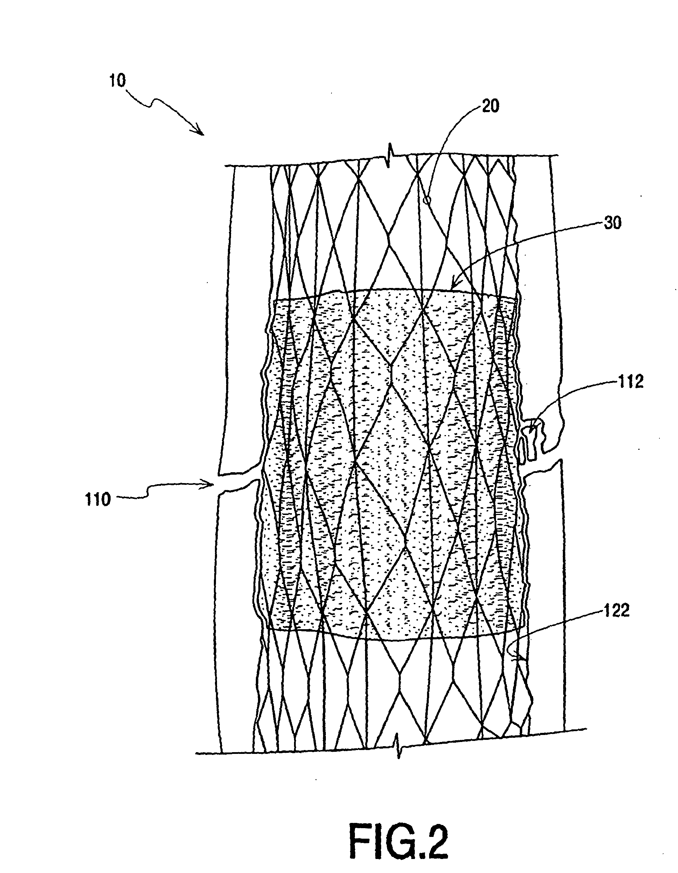

Figure 2 is a closer illustration of the fracture site 110 and the system 10. For illustration purposes, the space between the adjoining bone ends has been enlarged. As shown, the structural frame 20 may be configured to press against or otherwise engage the interior endosteal surface 122 of the bone 100. The structural frame 20 during its placement and expansion may assist in moving or aligning one or more bone fragments 112 that may have crushed into or otherwise encroached upon the intramedullary canal 120. Re-integration of bone fragments 112 is often an important aspect of the fracture healing process. In this aspect, the introduction of the expanding system 10 may aid in the reduction of the fracture by exerting outward forces and pressure from within the intramedullary canal 120.

As shown in Figure 2, the sheath 30 may be positioned to span the fracture site 110. Among other functions, the sheath 30 may prevent the surgical fluid 40 (not shown) from seeping into the fracture site 110. The sheath 30, together with the expanding structural frame 20 and the filling column of surgical fluid 40, may also facilitate the movement or alignment of one or more bone fragments 112 toward a more beneficial location.

Other embodiments of the system 10 may be possible, as described and shown herein. Although the system 10 illustrated in Figure 1 and Figure 2 includes a combination of structures, it is contemplated that the system 10 may comprise any one or more of such structures, either individually or in combination with other structures. Further, such structure or combination of structures may include any number of structures in any arrangement such as, for example, in a nested, overlapping, end-to-end, side-to-side, or other arrangement which provides for engagement and strengthening of the structures.

### The Structural Frame

By structural frame, it is meant that the structure may be configured to provide support to the bone and/or may facilitate the redistribution of forces acting upon the bone and fracture site, in order to provide stability and facilitate healing of the fracture. The structural frame may be configured to resist or redistribute compressive, tensile, torsional, bending, and shear forces exerted upon the bone and/or the fracture site.

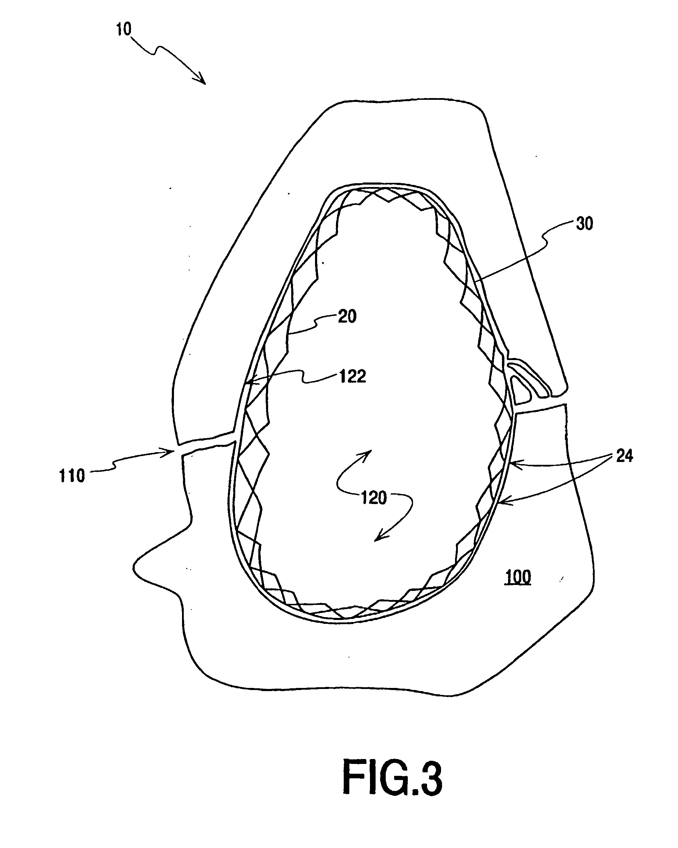

By way of example and not limitation, the structural frame 20 illustrated in Figure 1 may be shaped like a partially hollow, generally cylindrical tube, and it may be made of a mesh-like material. Other lengths, shapes, and densities are possible, so the structural frame 20 is not intended to be limited to the particular structures shown and described. For example, the structural frame 20 may comprise a generally elongate body having a generally hollow shape, which is substantially tubular in cross section, such as generally shown in Figure 3. The structure frame 20 comprises a cross-sectional size and shape suitable for insertion into an intramedullary canal 120 of a bone, as shown in Figure 3. The cross-sectional shape may be circular, triangular, rectangular, irregular, or some combination thereof, and may vary, at least in part, based upon the cross-sectional shapes of the canal 120 spanning and adjacent a fracture site 110.

As illustrated in Figure 1, the structural frame 20 may be constructed of a mesh-like material. The structural frame 20 may include one or more layers of fabric, coil or windings, in various shapes including helical, spiral, sinusoidal, linear variations, random patterns, or combinations thereof. In one embodiment, the structural frame 20 may be constructed of a mesh, matrix, lattice, or other configuration that allows for expansion. The material may be a metal, a plastic, or any other suitable material for use as an implant. In one embodiment, the material may be generally deformable but also capable of retaining its expanded shape after placement. Possible metals include stainless steel alloys, titanium alloys, nickel-titanium alloys such as Nitinol, cobalt alloys, magnesium alloys, and the like. Possible plastics include polyethylenes, polypropylenes, polyacrylates, fluorocarbons, silicones, polyacetals, polysulfones, polycarbonates, and the like. The structural frame 20 may be made of a bioabsorbable material, such as the materials used for absorbable sutures, and the frame 20 itself may contain antibiotics or other pharmaceuticals.

The structural frame 20 may be constructed of a single piece of material, or it may be constructed of a number of pieces nested together, interlaced, linked, hinged, or otherwise joined into a cooperative frame 20. A multi-piece structural frame 20 may include pieces having widely different shapes, properties, materials, and characteristics. Several embodiments for the frame 20 are discussed herein.

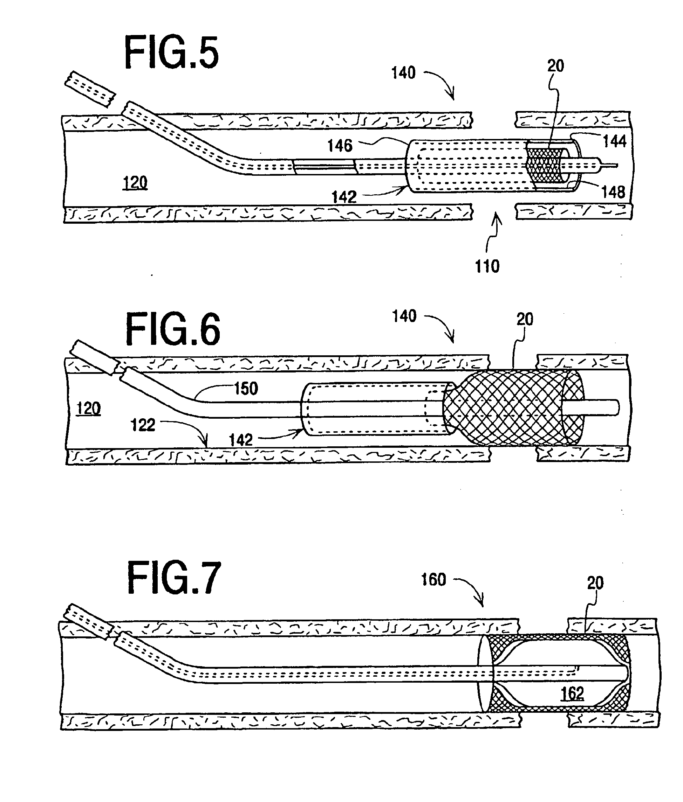

Expanding: The structural frame 20 may be expandable to allow it to be inserted in a collapsed state and then opened into an expanded state within the canal 120. The structural frame 20 may be constructed such that it is biased to open when released, as illustrated, for example, in Figures 5, 6, 8, and 9. The structural frame 20 may also be opened by force, for example, by an inflatable balloon or interior diaphragm, as illustrated in Figure 7.

Locking: In one embodiment, the structural frame 20 may be designed to lock into place once expanded into its final desired shape, such that the expanded structural frame 20 may resist the forces exerted upon it which may tend to collapse it. The locking aspect of the structural frame 20 may be accomplished by providing an overall mesh design that resists collapse once expanded. Alternatively, the structural frame 20 may include an additional integrated elements, such as one or more locking rings, positioned at critical locations where a resistance to collapse is desired.

Collapsing: The structural frame 20 may also be configured to expand to fill the canal 120, lock in place during healing, and collapse for later removal. A the method or tools used to expand the frame 20 may include a method or tool for later collapsing the frame a is smaller size, for removal from the canal 120.

As shown in Figure 2 and Figure 3, the structural frame 20 may be sized and shaped to expand with sufficient force to facilitate the movement and re-positioning of one or more bone fragments 112 that have crushed into or otherwise encroached upon the intramedullary canal 120. For example, when the mechanism of injury is a crushing force, and one or more bone fragments 112 are pushed into the intramedullary canal 120, the structural frame 20 in one embodiment may be used to drive such bone fragments 112 radially outward and generally toward a position more aligned with the opposing fracture ends, thereby promoting re-integration of the fragments 112 during the fracture healing process. In this aspect, the structural frame 20 may act like a stent inside the intramedullary canal 120.

The structural frame 20 may be sized and shaped to expand to fill gaps or voids in the cortical bone wall or endosteum 122 at or near the fracture site 110. For example, if one or more bone fragments 112 have separatad away from the fracture site 110 and are not compressed, aligned, or otherwise brought back nearer the fracture site 110, the structural frame 20 of the present invention may expand to fill the space once occupied by an absent fragment, In this aspect, an expandable and flexible structural frame 20 may fill the fracture site 110 beyond the space of the intramedullary canal 120 and offer still further support

In Figures 5 and 6, an apparatus, generally indicated at 140, comprises a structural frame 20 that may be configured to provide support to the fracture site without requiring additional structures such as for example, the sheath 30 and surgical fluid 40. The structural frame 20 shown in Figures 5 and 6 may be comprised of the any of the previous described materials; constructions, or patterns, and is preferably adapted so be self-expanding in that the material has shape-memory characteristics that allow the frame 20 to normally move toward an expanded cross-sectional size and shape when not being acted upon by a retainer or other structures.

By way of example, a retainer 142 is shown in Figures 5 and 6 for holding the frame 20 at a first cross-sectional size that is generally suitable during insertion of the frame 20 into the intramedullary canal 120 and during movement of the frame 20 toward the fracture site 110. The retainer 142 comprises a distal end 144, a proximal end 146, and an interior surface 148 for engaging the structural frame 20 in a collapsed position, as shown in Figure 5. The apparatus 140 may also include a delivery instrument, generally indicated at 150, which comprises a distal end 152 and a proximal end 154, and may further include a guide wire 156, as shown in Figure 37.

In Figure 6, the retainer 142 may be withdrawn in a generally proximal direction while the frame 20 remains at the fracture site 110. The frame 20 that emerges from inside the retainer 142 may begin to expand, due to its shape memory or biased characteristics, into a second cross-sectional size which is larger than the first cross-sectional size. The exterior surface of the frame 20 expands to engage the interior endosteal surface 122 of the intramedullary canal 120.

In Figure 7, an alternate apparatus, generally indicated at 160, comprises a structural frame 20 and an expandable member 162 positioned therein instead of the shape-memory characteristic employed in other aspects. The expandable member 162 may be employed at the distal end of the delivery member 150, catheter, guide wire, or other instrument. During insertion into the canal 120, the frame 20 is preferably positioned over the expandable member 140 to receive the expandable member 162 therein. In Figure 7, the expandable member 164 is expanded once the frame 20 is positioned at the fracture site 110. In Figure 7, the expandable member 162 may be a balloon which is operatively connected by an opening 166 to an inflation source (not shown) via an inflation lumen 164 defined in the deliver member 150. Alternatively, the expandable member 140 may be expanded by employing various techniques, such as, for example, articulation members, pull rods, control knobs, biasing members, or materials that exhibit their own shape memory characteristics.

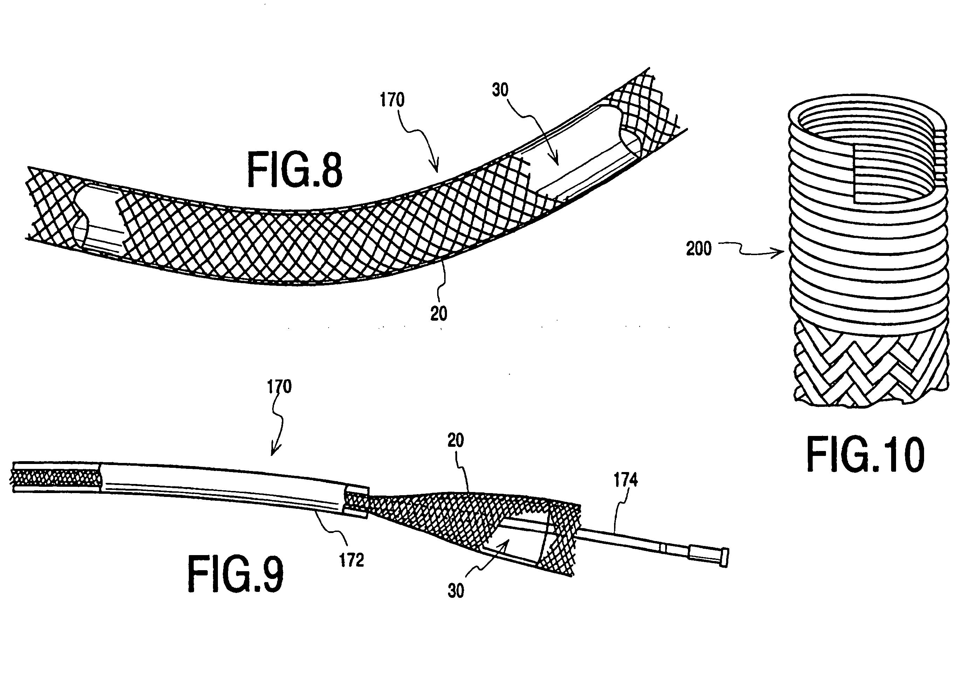

In Figures 8 and 9, an alternate apparatus, generally indicated at 170, which may be comprised of the structural frame 20 and sheath 30, as previously described and, as such, identical numbers will be used to identify these structures, except that in Figures 8 and 9, the sheath 30 is shown as being positioned within the interior of the structural frame 20. As shown in Figure 9, the structural frame 20 is preferably made of a material which is self-expanding such that the structural framework normally expands when it is not restrained by a retainer, generally indicated at 172.

In Figure 9, the retainer 172 may have a generally rigid, hollow, tubular, or cylindrical shape of fixed cross section to receive the structural frame apparatus 170 therein. The retainer 172 preferably holds the framework at a first cross-sectional size, which is generally smaller than a second cross-sectional size, such as, for example, during placement of the structural frame apparatus 170 into the canal 120 and toward the fracture site 110. The retainer 172 may be withdrawn or moved in a proximal direction to allow the structural frame apparatus 170 to expand to its second, expanded cross-sectional size. In Figure 9, both the structural frame apparatus 170 and the retainer 172 are shown with a guide facility 174 inserted through the hollow interior with an end of the guide facility extending from each end thereof which may be used to assist in positioning of the frame 170, as described in other aspects of the invention.

Figure 10 shows a structural frame 20, generally indicated at 200. The structural frame apparatus 200 may be comprised of a composite or combination of more than one material and/or construction to serve different functions. As shown in Figure 10, the structural frame apparatus 200 may be comprised of at least one first portion 202 of a fabric, mesh, lattice, matrix, or the like, which preferably aids or allows expansion of the structural frame apparatus 200, and is also comprised of at least one second portion 204 of a coil, winding, or spiral, which preferably aids flexibility or curvature of the structural frame apparatus 200. The portions 202, 204 may be positioned in an alternate repeating pattern of the same or different lengths or, alternately, the portions 202, 204 may be positioned at selected locations along the length of the structural frame apparatus 200. Other patterns will be apparent and may depend on the path which must be navigated by the structural frame apparatus 200 during insertion and placement.

Periprosthetic Fracture Fixation: In one embodiment, the system 10 of the present invention may be useful in fixing and stabilizing periprosthetic fractures. As shown in Figure 11A, a periprosthetic fracture 510 occurs near or around a prosthesis 500. Such fractures are increasing in frequency and are generally difficult to fix and stabilize. Because the prosthesis 500 is present in the intramedullary canal 120, the technique of intramedullary nailing is generally not an option. The bone 100 shown in Figure 11A is a human femur. The periprosthetic fracture 510 site, as shown, occurred near the distal end of the stem of a hip replacement prosthesis 500.

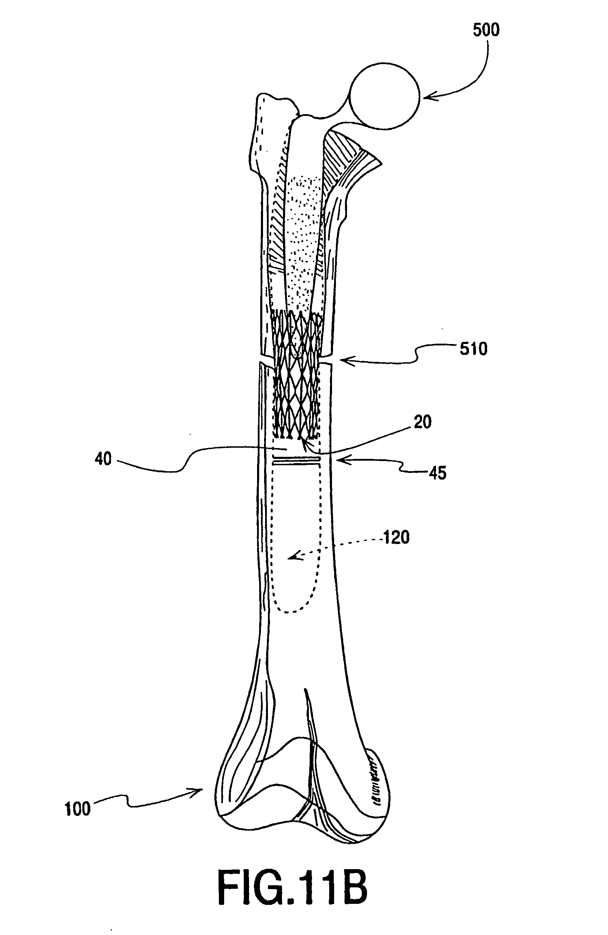

Figure 11B illustrates a structural frame 20, placed across a periprosthetic fracture site 510. The system illustrated may or may not include a sheath 30 spanning the periprosthetic fracture site 510. The structural frame 20, as shown in Figure 11 B, may extend beyond the distal end of the prosthesis 500. The canal 120 above the restrictor 45 shown may be filled with a surgical fluid 40. If the surgical fluid 40 surrounds both the frame 20 and a portion of the prosthesis 500, the hardened fluid 40 may provide sufficient strength and curability to stabilize the periprosthetic fracture site 510.

The structural frame 20 may be configured to envelop, surround, or otherwise engage the free end or stem of a prosthesis 500. The structural frame 20 may include additional elements or links specifically designed to connect the frame 20 to a particular prosthesis 500, to provide increased stability. In this aspect, the structural frame 20 may act as a kind of extension of the prosthesis 500, thereby extending the strength and reach of the prosthesis 500 beyond its original length and across the fracture site 510. The system 10 illustrated in Figure 11B may involve installation procedures that are somewhat different, of course, from those explained herein for fracture sites in bones where no prosthesis is present.

Figures 12-15 illustrate a further embodiment of a structural frame 20, generally indicated at 210. The structural framework 210 may be generally comprised of a plurality of elongated members 212, which may be positioned in a substantially circular configuration, as shown, although other configurations are also possible and may depend in part on the shape of the canal into which the structural framework 210 is inserted. As shown in Figure 12 and Figure 13, each individual elongated member 212 may be solid in cross-section, although other cross sections are also possible such as a hollow tube, shaft, or other structure. In addition, the elongated members 212 may have a cylindrical cross-sectional shape and other cross-sectional shapes are also possible.

In Figures 12-13, the structural framework 210 further comprises an elastomeric member, generally indicated at 214, which laterally extends between at least a portion of adjacent elongated members 212. By way of example, one elastomeric member 214 is shown, extending between the ends of two elongate members 212, although any number of such members 214 may be disposed along the length of the structural framework 210. As shown in Figure 12, a linking section 216 may be folded over or otherwise collapsed when the structural framework 210 is in an unexpanded position. In this regard, the elastomeric members 214 are preferably comprised of a resilient material which allows the elongated members 212 to be moved toward or away from each other, between unexpanded and expanded positions, as shown in Figures 12 and 13. In accordance with other disclosed aspects, the structural framework 210 may have self-expanding characteristics or may be opened using an expandable member such as a balloon.

By way of example and not limitation, the elastomeric member 214 in Figure 13 may be comprised of a linking section 216 and two sleeves 218 disposed on each end of the linking section 216. Each sleeve 218 may receive a separate elongated member 212 or may be otherwise connected thereto, such as with a snap-together or interference fit or using mechanical fasteners. Such members 214 or any one of their components may be comprised of the same or different materials as the material which comprises the remainder of the structural framework 210. Alternatively, the elastomeric member 214 may also be comprised of a spring, coil, or other appropriate structure to allow expansion or biasing of the elongated members 212 away from one another.

As shown in Figures 14 and 15, the structural framework 210 may be inserted into an intramedullary canal of a bone 100 having a fracture site while in an unexpanded condition, as illustrated in Figure 14. The framework 210 may be expandable, as illustrated in cross-section in Figure 15, when the framework 210 has been properly positioned within the intramedullary canal 120.

Figures 16 and 17 illustrate a structural frame 20, generally indicated at 230. The structural framework 230 is similar to the embodiment (210) shown in Figures 12-15, in that the structural framework 230 of Figures 16 and 17 comprises elongated members 232 and at least one elastomeric member 234 which extends between adjacent elongated members 232 at a location near the distal ends. Figures 16-17 illustrate elastomeric members 234 that are formed as an integral part of the structural framework 230, with each end of such member 234 connected to the elongated members 232 at a junction, although other constructions are possible. Figures 16-17 further illustrate elongated members 232 having a generally triangular shape. Other shapes of the elongated members 232 are possible, such as discussed herein.

Figures 18-20 illustrate a structural frame 20, generally indicated at 240. The structural framework 240 may be made of any material which preferably allows expansion of the framework 240 either by an expansion member or by the shape memory characteristics of the structural framework 240 itself. Similar to the embodiment (210) shown in Figures 12-17, the structural framework 240 illustrated in Figures 18-20 comprises elongated members 242 which extend along the framework 240, as shown in Figure 18. The framework 240 further comprises a plurality of linking members 244 which extend between adjacent elongated members 242, spaced along the length of the framework 240, as shown in Figure 18. When the framework 240 expands, as shown in diagrammatic view in Figure 18, the connections or junctions between the elongated members 242 and the linking members 244 allow flexing or bending, in order to accommodate expansion of the framework 240, thus allowing the lateral distance between adjacent members 242 to increase.

In Figures 21-23, another structural frame 20 is illustrated, generally indicated at 250, which shows an alternative linking arrangement for permitting expansion of the structural framework 250. The structural framework 250 may be comprised of a first elongated member 252 and a plurality of second elongated members 254. In Figure 22, the first elongated member 252 may have a hollow tubular shape which extends along a longitudinal axis 253 between distal and proximal ends of the framework 250, as shown in Figure 21. The first elongated member 252 may be comprised of a wire, coil, tube, or other like material, or a combination of such materials. As shown in Figure 22, the plurality of second elongated members 254 preferably are spaced radially outward from the first elongated member 252, and are laterally spaced apart from one another.

In Figure 22, the plurality of second elongated members 254 are shown longitudinally offset from the first elongated member 252 at the proximal end of the framework when the framework 250 is disposed in an unexpanded position. Although other lengthwise configurations are possible, the first and second elongated members 252, 254 are preferably similar in longitudinal extent such that the distal end (not shown) opposite the proximal end in Figure 22 are also offset. Each of the first and second elongated members 252, 254 may be comprised of any of the materials described herein.

In Figures 21-23, a plurality of linking members 256 may be disposed about the first elongated member 252 in order to connect it to the plurality of second elongated members 254. As shown in Figure 21, the linking members 256 may be spaced around the first elongated member 252 at one or more locations along the length of the framework 250 between the distal and proximal ends thereof. In Figure 22, each linking member 256 is preferably connected by a first pivot or hinge 258 at one end to the first elongated member 252 and connected by a second pivot or hinge 260 at another end of one of the second elongated members 254. The linking members 256 may be a wire, tape, string, spring, chain, or other like member. As shown in Figure 22, the individual linking members 256 may be winded or twisted from a single material which engages the hinges 258, 260 or, alternatively, the linking member 256 may be discrete pieces of material that are disposed between the hinges 258, 260.

As shown in Figures 22 and 23, the linking members 256 may be configured to allow relative movement between the first and second elongated members 252, 254 during expansion of the framework 250 between the non-expanded position (shown in Figure 22) and the expanded position (shown in Figure 23). As the framework 250 expands to the position shown in Figure 23, the first elongated member 252 is moved downward or in a distal direction (or the second elongated members 254 move upward or in a proximal direction). The linking members 256 also pivotably move at the pivots 258, 260 to accommodate the relative movement between the first and second elongated members 252, 254. Such pivotal movement of the linking members 256 allows the first and second elongated members 252, 254 to be disposed such that they are relatively aligned longitudinally at their respective ends, as shown in Figures 21 and 23, and they are laterally separated by the linking member 256 which is laterally disposed between the first and second members 252, 254. The framework 250 may be returned or reversed to its unexpanded position (shown in Figure 22) by moving the first elongated member 252 in an upward or proximal direction (or by moving the second elongated members 254 in a downward or distal direction) relative to Figure 23.

In Figures 24-27, an alternate structural framework 270 is illustrated, which includes a first elongated member 272 and a plurality of second elongated members 274. The structural framework 270 is similar in structure and movement to the embodiment (250) illustrated in Figures 21-23, except that the framework 270 preferably includes a plurality of cross members 276 which are connected between adjacent second elongated members 274 at connection regions 278 along the length of the framework 270. Although the cross members 276 may have any shape or configuration, they are shown in Figures 24-27, as having a V-shape, and an inverted V-shape in alternating configuration. The ends of adjacent shapes are joined together and the vertices are slightly spaced apart along the length of the framework 270 to permit expansion of the framework 270. It is contemplated that other shapes and configurations are also possible and are not limited to the shapes and configurations shown herein.

Like the embodiment (250) in Figures 21-23, the alternate structural framework 270 shown in Figures 24-27 may be moved from the unexpanded position (shown in Figures 24 and 26) to an expanded position (shown in Figures 25 and 27) as described above. Such expansion permits relative movement between the first and second elongated members 272, 274 and pivotal movement of linking members 280, each end of which is pivotably connected between the first and second elongated members 272, 274.

Prongs: As shown in Figure 3, one modification of the structural frame 20 described herein may also include one or many small prongs 24 or gripping members disposed on or along the outer surface of the structural frame 20, in order to provide a secure attachment to the inside walls of the intramedullary canal 120. The endosteum 122 is a layer of vascular connective tissue lining the medullary cavities of bone. As illustrated in Figure 3, the prongs 24 of the structural frame 20 may be shaped to engage the endosteal surface 122 of the bone 100, such that the structural frame 20 remains in place inside the intramedullary canal 120.

The endosteal surface 122 is generally rough in texture, allowing any of a variety of prong shapes and sizes to be used. The prongs 24 may be formed by the intersecting edges of the mesh of the structural frame 20 itself, as depicted in Figure 3, or the prongs 24 may be additional structures interwoven or otherwise attached or integrated into the structural frame 20. In one embodiment, the prongs 24 may be located throughout the surface of the structural frame 20. The prongs 24 may be located only on the exposed end portions of the structural frame 20 which are not covered by the flexible sheath 30. The prongs 24 may be provided on a separate element positioned within or around the structural frame 20 at the desired location for an efficient attachment. In general, the prongs 24 may be configured to provide a firm and stable grasp of the endosteal surface 122 such that the structural frame 20, once in place, can withstand the expected biomechanical forces exerted across the fracture site without moving or failing.

Strain Gage System: The system 10 may include a strain gage system to measure movement or deflection around the fracture site 110. The strain gage system may include a strain gage positioned on or near the structural frame 20 and across the fracture site, a transmitter, and a receiver. By "across the fracture site," it is meant that the strain gage may be positioned with a first end adjacent or near a first bone end, and a second end near the opposing second bone end. The transmitter may be wireless and it may be installed within the bone or the body of the patient. The receiver may also be wireless. The strain gage system may be useful to sense the relative movement between the bone ends. Decreasing relative motion would indicate progress in healing, as the bone ends reconnect. A patient may be released to return to normal activity when the relative motion decreases to a certain pre-determined limit. Also, a patient may be restricted in activity if the strain gage indicates relative motion above a certain limit.

Electrical Stimulation: The system 10 may include an electrical osteogenic stimulator to promote bone union and healing, in and around the fracture site 110. The stimulator may be implantable, and may include an electrode connected directly to the structural frame 20 so that a current is delivered directly or perpendicular to the fracture. The stimulator may be non-invasive, and may include a cuff worn outside the body near or around the fracture site 110.

### The Sheath

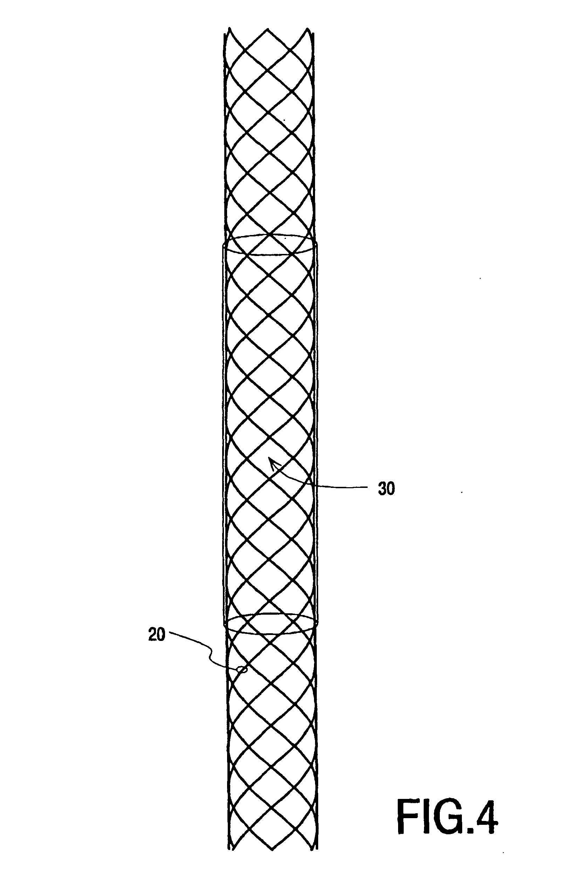

Turning back to Figures 1-4 generally the present invention described herein includes the flexible sheath 30, Figure 4 is a perspective illustration of a sheath 30 positioned around a structural frame 20. The structural frame 20 and the sheath 30 each have properties and advantages in the absence of the other, or alternatively, in combination with each other, and these structures may find applicability either alone or in combination.

As shown in Figure 1, the sheath 30 finds applicability in combination with the structural frame 20 and may cover a substantial portion of the length of the structural frame 20. The sheath 30 may be generally flexible, pliable, and deformable, such that when filled it takes the shape of the interior space where it is placed. The sheath 30 may be constructed of collagen, polyester fabric such as Dacron®, polylactide (PLA) polymer fibers, or any other suitable elastic, biologically inert material. The sheath 30 may be permanent or it may be made of a bioabsorbable material The sheath 30 may be made of a single layer or multiple layers.

The fabric or materiel of the sheath 30 may be knitted, woven braided, form-molded, of otherwise constructed to a desired porosity. The sheath 30 of the invention is made of a material defining a plurality of pores, said pore size selected to be substantially impermeable to a surgical fluid 40, thereby reducing intrusion of said fluid 40 into the fracture site 110. In one embodiment, the porosity of the sheath 30 will allow the ingress and egress of fluids and solutions, and will allow the intrusive growth of blood vessels, fibrous tissue, bony trabeculae, and the like, while the porosity is low enough so the sheath 30 may retain small particles of enclosed material such as a surgical fluid 40 or cement, a biological mediator 50, or other materials known to promote bone formation, healing, or general bons health. The fabric defines a plurality of pores. The pore size may be selected to allow tissue growth through and around the sheath 30 while also containing the material injected or otherwise packed into the sheath 30.

The sheath 30 may be coated or otherwise infused with a biological mediator 50. The future of fracture healing may frequently involve the delivery of a biological mediator 50 directly to the fracture site. The mediator 50 may include genetically altered cells, cytokines, bone graft or bone graft substitutes, bone morphogenic proteins, hydroxyapatite, osteoblasts, osteogenic agents such as bone marrow stomal cells, stem cells, or other precursors to bone formation, artificial biocompatible or biological chemicals or materials, such as octeoconductive matrices or other osteoinductive chemicals. The term biocompatible is meant to include materials or chemicals which are osteoconductive in that such materials or chemicals may allow bone growth or permit bone growth without obstruction, or which are osteoinductive in that such materials or chemicals induce, stimulate, or otherwise promote bone growth. Other materials such as antibiotics or other pharmaceuticals may also be beneficial if delivered directly to the fracture site without disruption of the biology.

In another embodiment, the sheath 30 may act as a three-dimensional culture matrix for a biological mediator 50 to be delivered directly to the fracture site 110.

### The Surgical Fluid

The surgical fluid 40 may be a polymer bone cement such as PMMA (polymethyl methacrylate), calcium phosphate cement, a bone graft substitute, a collagen matrix colloid, or any other material that provides sufficient strength upon hardening. The fluid 40 may be bioabsorbable or not, and it may contain antibiotics or other pharmaceuticals.

In general, the surgical fluid 40 may be selected and inserted in order to form a hardened column spanning the fracture site 110, as shown in Figure 1. The hardened surgical fluid 40 may partially surround and otherwise attach to the structural frame 20 and the endosteal surface 122 of the bone, thereby providing a support structure for the bone. One function of the hardened surgical fluid 40 is to transfer the expected biomechanical forces on the bone and transfer these forces from the bone 100 to the structural frame 20, thereby providing support and stability to the fracture site 110 during the healing process. Another function of the hardened surgical fluid 40 is to provide support to the structural frame 20 itself by preventing buckling or lateral movement of the components of the structural frame 20 when the expected biomechanical forces are applied.

The surgical fluid 40 may be a non-absorbable PMMA product, such as Surgical Simplex P, Palacos^{®} R, Zimmer Regular, Zimmer Low Viscosity (LVC), CMW-1, CMW-3, Osteopal^{®}, Osteobond^{®}, Endurance^{™} bone cement, or a similar product. The surgical fluid 40 may be a non-absorbable PMMA product with antibiotics, such as Palacos^{®} R with gentamycin, Surgical Simplex P with tobramycin, or a similar product. The surgical fluid 40 may be an absorbable product, such as Norian SRS^{®}, calcium phosphate cement (CPC), calcium phosphate hydraulic cement (CPHC), sodium citrate modified calcium phosphate cement, hydroxyapatite (HA) cement, hydroxyapatite calcium phosphate cements (CPCs); a beta-TCP-MCPM-CSH cement [beta-tricalcium phosphate (beta-TCP), monocalcium phosphate monohydrate (MCPM), and calcium sulfate hemihydrate (CSH)]; a bioactive bone cement (GBC) with bioactive MgO-CaO-SiO2-P2O5-CaF2 glass beads and high-molecular-weight polymethyl methacrylate (hPMMA); a tricalcium phosphate (TCP), tetracalcium phosphate (TTCT), and dicalcium phosphate dehydrate (DCPD) bone cement with dense TCP granules; an hPMMA with delta- or alpha-alumina powder (delta-APC or alpha-APC); a similar product; or any other material that provides sufficient strength upon hardening.

The Surgical fluid 40 may be introduced into the intramedullary canal 120 in its least-viscous state and allowed to generally fill the space inside the structural frame 20 and may infuse into or otherwise encompass the fabric or mesh of the structural frame 20. The fluid 40 may also interdigitates into the endosteal bone. The surgical fluid 40 may be contained by the sheath 30 at the fracture site while the fluid cures. During curing, however, the fluid 40 may be slightly deformable so that the pressure variation within or exerted upon the fluid 40 will produce a desired amount of flow through and around the structural frame 20. Once cured, the fluid 40 may form a hardened column that contains the structural flame 20 as a kind of reinforcing cage, adding support and stability to the hardened column. The sheath 30, in one embodiment, may surround a portion of the hardened column that spans the fracture site 110.

An embodiment may include a vibration probe 60 to remove any air voids from the surgical fluid 40 as it begins to cure. Removal of air using a vibrating probe 60 may provide improved interdigitation of the cement column, both proximally and distally, for better resistance to torsional stresses that may be exerted near the fracture site. The fluid 40 may be pressurized to remove air voids and improve interdigitation. The surgical fluid 40 itself may be modified to include compounds or additives that improve its biomechanical strength and durability when hardened.

### The Fluid Restrictor

As shown in Figure 1, the system 10 may include a fluid restrictor 45 positioned within the intramedullary canal 120 to support a quantity of surgical fluid 40. For a bone in a generally vertical posture, as shown, the restrictor 45 may be referred to as a base and the quantity of surgical fluid 40 may be referred to as a column of surgical fluid 40. In an alternative embodiment, the fluid 40 may be allowed to generally fill the intramedullary canal 120 without the presence of any restrictor 45.

The restrictor 45 may be generally cylindrical, as shown in Figure 39, or it may take any other shape appropriate to the particular bone or space to be filled. The restrictor 45 may be comprised of one or more generally annular concentric platforms. The restrictor 45 may be rigid or flexible. The generally cylindrical restrictor 45 illustrated in Figure 39 may be flexible so that it fits within a generally non-cylindrical intramedullary canal 120, such as the one illustrated in Figure 3. The restrictor 45 may be constructed from any of a variety of materials, including those described herein, and may be comprised of elastomeric material or hydrophilic material which expands when placed in a fluid such as water. The restrictor 45 may be bio-absorbable. The restrictor 45 may be permanent or temporary.

The restrictor 45 may be an inflatable balloon or diaphragm, held in place when inflated, until the surgical fluid 40 hardens to a viscosity sufficient to support itself within the canal, at which time the restrictor 45 may be collapsed and withdrawn. A balloon restrictor 45 may be collapsed for insertion, positioned at a desired location, and inflated with saline or air. The balloon material may permit the restrictor 45 to expand asymmetrically, so that when inflated it will fill a generally asymmetric intramedullary canal 120, such as the one illustrated in Figure 3. The balloon material may include a variety of panels or sections, perhaps of different materials, in order to accommodate a specific canal 120 having a particular shape. After the restrictor 45 has been positioned and inflated, the surgical fluid 40 or cement may be injected to the region. After the surgical fluid 40 hardens to a sufficient viscosity, the restrictor 45 may be drained or otherwise collapsed and withdrawn.

In one embodiment, the restrictor 45 may be attached to or formed as an integral part of the sheath 30, so that the restrictor 45 and sheath 30 together form a generally open container with the restrictor 45 as the base or bottom.

### The Guidance Instruments

As shown in Figure 37, the system 10 may include a guide wire 156, a delivery instrument 150, and a retrieval tool 290 to facilitate the manipulation of the structural frame 20.

The guide wire 156 may be placed in the intramedullary canal 120 and used for guidance during installation of the various elements of the inventive system 10. The guide wire 156 may be generally flexible in order to facilitate insertion and manipulation. The guide wire 156 may be a wire, tape, tube, shaft, or other like elongate structure. The guide wire 156 may be approximately three millimeters in diameter. The guide wire 156 may include one or more hinged sections positioned at intervals along its length to facilitate bending or articulation at certain points where increased elasticity is desired.

The guide wire 156 may comprise a proximal end and a generally opposing distal end. The proximal end may include a handle or may be otherwise graspable. The distal end may include a ball 157 disposed on the tip, as shown in Figure 38, to drive or otherwise facilitate the movement and placement of the restrictor 45. The restrictor 45, as shown in Figure 39, may include a depression or seat 46 sized and shaped to receive the ball 157. The restrictor 45 may also include a hole 47 sized and shaped to receive both the ball 157 and a portion of the distal end of the guide wire 156.

The distal end of the guide wire 156, as shown in Figure 38, may also include on or more fins 158 sized and shaped to effect the expansion of the structural frame 20, as explained in more detail below.

The delivery instrument 150, as shown in Figure 37 and 41, may be used to facilitate the manipulation of the structural frame 20. The delivery instrument 150 may comprise a proximal end 154 and a generally opposing distal end 152. The proximal end 154 may include a handle or may be otherwise graspable. The distal end 152 may be sized and shaped to engage and push one end of the structural frame 20. The delivery instrument 150 may define a generally central opening along its length, so that the delivery instrument 150 may be placed over the guide wire 156. With the guide wire 156 passing through the generally central opening, the delivery instrument 150 and the structural frame 20 may be guided into the canal for installation.

Expansion by Fins: Figure 41 illustrates the structural frame 20 in a generally collapsed condition. Figure 43 illustrates the structural frame 20 approaching the distal end of the guide wire 156 and the one or more fins 158 disposed thereon. The one or more fins 158 are sized and shaped to open the structural frame 20 when the delivery instrument 150 is used to execute a final push of the frame 20 toward the restrictor 45. As shown in Figure 43 and Figure 44, the fins 158 may engage a generally interior portion of the structural frame 20 such that the frame 20 is forced toward its generally expanded condition when the frame 20 moves toward the restrictor 45. Figure 42 illustrates the structural frame 20 in a generally expanded condition.

Expansion by Articulated Section: Figure 41 illustrates the structural frame 20 in a generally collapsed condition. Figure 45 illustrates the proximal or upper end of the structural frame 20 at or near its installed position. The structural frame 20 may include an articulated section 25 positioned at or near the proximal or upper end of the frame 20 and sized and shaped to open the structural frame 20 when the delivery instrument 150 is used to execute a final downward push of the frame 20. As shown in Figure 45 and Figure 46, the distal end 152 of the delivery instrument 150 may engage the articulated section 25 such that, when a force is applied by the delivery instrument 150, the various structural members of the articulated section 25 drive the frame 20 open, toward its generally expanded condition. Figure 46 and Figure 42 illustrate the structural frame 20 in a generally expanded condition.

The retrieval tool 290, as shown in Figure 37 and Figure 48, may also be used to facilitate the manipulation of the structural frame 20. The retrieval tool 290 may comprise a proximal end and a generally opposing distal end. The proximal end may include a handle or may be otherwise graspable. The distal end may include a hook 292 disposed on the tip. The hook 292 may be used to engage the structural frame 20 or one or more other elements of the inventive system 10. The hook 292 may be used, in one embodiment, to engage and remove the structural frame 20 or one or more other elements from the canal 120.

Alternative Tools: As shown in Figures 28-33, the system 10 may include an installation tool 300, a Y connector 310, an inflation instrument 320, and a syringe 300, to facilitate the manipulation of the structural frame 20.

As shown in Figures 28-29, the structural frame 20 may be placed on the distal end of the installation tool 300, and together they may be placed into the intramedullary canal 120. The tool 300 may be used for guidance during installation of the various elements of the inventive system 10. The tool 300 may be generally flexible in order to facilitate insertion and manipulation, and it may be hollow. The tool 300 may be a tube, shaft, wire, or other like elongate structure.

The tool 300 may comprise a proximal end and a generally opposing distal end. The proximal end may include a handle or may be otherwise graspable. As shown in Figures 28-29, a Y connector 310 may be disposed on or attached to the proximal end. The distal end may have a blunt or rounded shape. The hollow portion in the tool 300 may germinate at the distal end.

As shown in Figures 29-30, an inflation tool 320 may be used to expand the structural frame 20 from it collapsed condition (Figure 29) to its expanded condition (Figure 30). The inflation tool 320 may connect to a first inlet of the Y connector 310, as shown.

As shown in Figures 31-32, a fluid injection device or syringe 330 may be used to inject surgical fluid 40 into the intramedullary canal 120. The syringe 330 may connect to a second inlet of the Y connector 310, as shown. In the embodiment illustrated here, there is no restrictor 45. Instead, the surgical fluid 40 fills the lower portion of the canal 120. Figures 31-32 illustrate a retrograde injection of surgical fluid 40, which means the injection begins at the generally distal end of the canal 120 and proceeds toward the proximal end. As shown in Figure 32, the syringe 300 and the installation tool 300 may be withdrawn as the retrograde injection progresses. Figure 33 shows the column of surgical fluid 40 after injection.

### Illustrative Use of the System

The maneuvers and manipulations to be performed, as well as the size and shape of the structural frame 20 to be used, are desirably selected by a medical professional (such as a physician, surgeon, physician's assistant, or other qualified health care provider), taking into account the morphology and geometry of the site to be treated. The shape of the bones, joints, and soft tissues involved, and the local structures that could be affected by such maneuvers and manipulations are generally understood by medical professionals using their expertise and their knowledge of the site and its disease or injury. The medical professional is also desirably able to select the desired shape and size of the structural frame 20 and its placement, based upon an analysis of the morphology of the affected bone using, for example, plain-film x-ray, fluoroscopic x-ray, MRI scan, CT scan, or the like, and templates that accurately size the implant to the image. The shape, size, and placement of the structural frame 20 and related elements of the system 10 are desirably selected to optimize the strength and ultimate bonding of the fracture relative to the surrounding bone and/or tissue.

A method may include a percutaenous (through the skin) surgical technique. Percutaneous techniques offer many advantages in orthopaedic surgery. Small incisions allow for decreased blood loss, decreased postoperative pain, decreased surgical time, and a shorter time under anesthesia for the patient. Also, rehabilitation is accelerated, hospital stays are shorter, and the fracture biology is preserved by eliminating extensive dissection at the fracture site. Minimally invasive techniques have been shown to provide an overall better result for most procedures, provided that they can be accomplished without undue risk to the patient.

As shown generally in Figure 1, the elements of the system 10 may be inserted or otherwise introduced into the intramedullary canal 120 of a fractured or diseased bone 100. The dash line in Figure 1 represents an insert path 90 that leads generally from a location external to the patient, through an incision 70 in the skin, through an opening or breach 80 in the bone, and along an approximate centerline through the intramedullary canal 120 toward the fracture site 110.

The technique or method may include a variety of instrumentation to create access to the intramedullary canal 120, including a scalpel and other cutting instruments to create an incision 70 and a channel through the other tissues between the skin and the bone, one or more drills and drill bits to breach the cortical bone and create a breach 80, and one or more cannulated delivery systems for passing instruments and elements of the system 10 along the insertion path 90 toward the fracture site 110. The various elements of the system 10 and the instrumentation may be radiopaque so the surgeon may accomplish the techniques under intraoperative fluoroscopic guidance.

The technique or method may include one or more flexible guide wires, such as the guide wire 156 shown in Figure 37, flexible delivery tubes or cannulae that are sized and shaped to pass an expandable balloon or diaphragm into the canal 120, and other cannulae sized and shaped to pass restrictors, structural frames 20, vibration probes, and other components of use to and from the fracture site 110. The surgical fluid 40 may be introduced at the fracture site 110 using a fluid injective device or syringe with a flexible hose and a nozzle sized and shaped to travel along the insertion path 90. The surgical fluid 40 may also be introduced through the same installation tool used for placing the structural frame 20, via a multi-lumen tubing with exports at the distal end or through the lumen used for the guide wire after the guide wire has been removed.

Insertion & Removal: Referring to the illustrations in Figure 1 and Figures 37-50, the method may generally include the execution of one or more of the following general steps by a user such as a medical professional.

In general, the medical professional may begin by locating the disease or fracture site 110, relative to known physical landmarks. Based upon the location of the fracture site 110, the medical professional may select a suitable location for placing the breach 80 into the bone and a corresponding site for the incision 70. In one embodiment, the medical professional may use an arthroscope to gain access to the intramedullary canal. Arthroscopy offers direct visualization of the interior of a joint or other cavity, and the fracture site. The arthroscope may be used and find a suitable location for placing the breach 80 into the bone, as well as to examine the fracture site 110 itself. Arthroscopic guidance may be an attractive tool for a variety of specific fracture types, particularly if in-line access to the medullary canal is desired and access to a joint at the proximal or distal aspect of the fractured bone is required.

The medical professional may make the incision 70 in the skin and locate the selected site for the breach 80. A drill may be used to create the breach 80 through the cortical bone and into the intramedullary canal 120. In one embodiment, the breach 80 may be approximately one-quarter inch in diameter and may be oriented at an angle of approximately forty-five degrees relative to the bone 100 as illustrated in Figure 1.

The breach 80 may be positioned to allow arthroscopic visualization of the intramedullary canal as well as the insertion of guidance tools, a structural frame 20, and related elements. In the femur, an access hole or breach 80 may be drilled in the femoral notch between the condyles. In the humerus, the access hole or breach 80 may be drilled through the humeral head. Arthroscopic insertion may be advantageous because it offers axial access (a straight path) into the intramedullary canal. With axial access, the structural frame 20 may be less flexible because it may be inserted along a substantially linear path. Access through the bone end, however, is generally not recommended for children because it may compromise the growth plate. Arthroscopic guidance, visualization, and insertion may be an attractive tool for a variety of specific fracture types, including those described above.

The system 10, as shown in Figure 37, may include a guide wire 156, a delivery instrument 150, and a retrieval tool 290 to facilitate the manipulation of the structural frame 20 of the present invention. The medical professional may insert the guide wire 156 alone into the intramedullary canal 120. Alternatively, the medical professional may insert the distal end of the guide wire 156 into or through a restrictor 45, and insert the combination into the intramedullary canal 120, as shown in Figure 40. The restrictor 45 may be preferably placed at a suitable location away from the fracture site 110.

The medical professional may select an apparatus for insertion which may be any one of the apparatuses described herein such as the structural frame 20, the apparatus 140, the alternate apparatus 160, the structure frame apparatus 200, the structural frameworks 210, 230, 250, 270. The apparatus to be inserted will be referred to as the structural frame 20. In general, the structural frame 20 selected is preferably sized and shaped to fit the size of the intramedullary canal 120 near the fracture site 110.

The medical professional may cover a select portion of the stuctural frame 20 with the sheath 30 or, alternatively, may insert the sheath 30 into a portion of the structural frame 20. The structural frame 20 may be supplied already covered with a sheath 30. The sheath 30 may be sized in length to span to fracture site 110. The location of the sheath 30 relative to the structural frame 20 may be estimated using the location of the fracture site 110, the length of the structural frame 20, and the expected position of the frame 20 when installed. When installed, the sheath 30 preferably spans the fracture site 110 as shown in Figure 1.

The medical professional may use a delivery instrument 150, as shown in Figure 37 and 41, to facilitate the manipulation of the structural frame 20. The guide wile 156 may bo inserted into the generally open central portion of the stuctural frame 20, so that the frame 20 may be moved generally toward the fracture site 110. Likewise, the guide wire 156 may be inserted into a generally open passage through the delivery instrument 150, so that the instrument 150 may also travel along the guide wire 156 toward the fracture site 110. The distal end 152 of the delivery instrument 150 may be used to push or otherwise manipulate the structural frame 20 into and along the intramedullary canal 120.

The medical professional may insert the structural frame 20 along the insertion path 90, assisted by the guide wire 156, toward the fracture site 110 until the structural frame 20 reaches the restrictor 45, as illustrated in Figure 41. The structural frame 20 may be positioned such that the sheath 30 spans the fracture site 110 and the unsheathed portion extends into the intramedullary canal 120 on opposing sides of the fracture site 110, as shown in Figure 1.

The step of expanding the structural frame 20 may be accomplished as described herein, including by removal of a retainer 142 (as shown in Figures 5 and 6) to allow a self-expanding frame 20 to expand, by inflating a balloon or diaphragm inside the frame 20, by using the delivery instrument 150 to push the structural frame 20 toward one or more fins 158 positioned near the distal end of the guide wire 154 (Figures 43 and 44), or by pushing the distal end of the delivery instrument 150 against an articulated section 25 positioned at or near the proximal or upper end of the frame 20 (Figures 45 and 46). In general, the structural frame 20 may be expanded until the one or more prongs 24, if provided thereon, engage the endosteal surface 122 as shown in Figure 3. The structural frame 20 may then be locked in its expanded position.

A retainer 142 may also be used to prevent the inadvertent expansion of a non-self-expanding structural frame 20, and to protect the frame 20, at all times other than when expansion is specifically desired.

Surgical fluid 40 may then be introduced by the medical professional into the intramedullary canal 120. The fill may begin at the foot of the canal 120 or at the base provided by the restrictor 45 if one is used. The surgical fluid 40 may be injected to completely fill the apparatus installed or only a portion thereof.

The medical professional may insert a vibrating probe along the insertion path 90 until the probe end is positioned within the body of surgical fluid 40. The vibrating probe may be used to remove any air voids from the surgical fluid 40 and agitate the fluid 40 to promote the laminar flow characteristics of the fluid and to promote interdigitation into and through the structural frame 20 and the surrounding endosteal surface.

The guide wire 156 may be removed or left in place permanently. The guide wire 156 may be cut, at or near the breach 80 or bone surface (as shown in Figure 47) or near the incision 70, and left in place, inside the canal 120. The breach 80 and/or the incision 70 may be closed. Temporary external stabilization may be provided while the surgical fluid 40 cures into a hardened column.

These general steps are provided as a broad description of the technique and steps of the method of installing the system 10. As will be appreciated by those skilled in the art of orthopaedic surgery, many additional or complementary steps may be performed, in various order, to accomplish any of a number of supplemental or supportive tasks as part of the technique or method.

Forces on the System: The system 10 may provide sufficient fixation, stabilization, and resistance to the expected biomechanical forces exerted across the fracture site during the healing phase that no external splinting or casting will be needed. The hardened column, together with the structural frame 20 and the prongs 24 engaging the endosteal surface 122, may be sufficient to withstand forces in compression and extension, torsion, and shear. In this aspect and in others, the present invention offers an alternative to external casting.

With respect to such forces, Figures 34-36 diagrammatically show several forces which may act upon the system 10. In general, the elements of the system 10 may assist in the transfer of forces across the fracture site 110 so that such forces are substantially resisted by the structural frame 20 and/or surgical fluid 40 or substantially diverted or transferred elsewhere. The system 10 minimizes the effect of such forces upon the fracture site 110 during the healing phase and facilitates recovery and bone growth. In Figures 34-36, the forces are depicted using arrows to indicate the direction in which the force is being applied.

In Figure 34, the force arrows shown are associated with a compressive force (squeezing) acting upon the bone having the fracture site 110. In Figure 34, the compressive load (top vertical arrow) is first transferred from the upper joint to the bone shaft. The load is then transferred from the bone shaft to the system 10 (*i.e*., the structural frame 20 and/or the hardened surgical fluid 40) via shear forces at the system-bone interface at a location generally proximal the fracture site 110. The surgical fluid 40 and the structural frame 20 preferably carry the compressive load across the fracture site 110, although some portion of the force may be transferred through the fracture site 110 due to contact with bone fragments in the vicinity. Below or distal the fracture site, the force is transferred back to the bone shaft through shear forces at the system-bone interface and finally transferred back through the lower joint.

Turning to Figure 35, a torsional force (twisting) is shown acting upon the bone. A minimal degree of resistance to torsion is provided at the fracture site 110 in the form of friction between the bone fragments. Torsional forces are transferred from the bone shaft to the system 10 by shear forces at the system-bone interface. Torsion is transferred across the fracture site 110 and the load is preferably carried by the structural frame 20 and/or the surgical fluid 40. Below or distal the fracture site in Figure 35, torsion is transferred back to the bone shaft via shear forces at the system-bone interface.

Turning now to Figure 36, the force arrows shown are associated with a lateral force (sideways) acting upon the bone. The system 10 may assist the fracture site 110 in bearing, distributing, diverting, or otherwise carrying the lateral force. When a lateral force is applied to the bone from the right (as shown), the force will tend to cause a bending of the bone. The lateral force induces a compressive force (squeezing) on the side where the lateral force is applied, and a tensile force (stretching) on the opposing side of the bone. Forces along the generally central longitudinal axis of the bone are typically minimal or zero. Bone fragments at or near the fracture site may resist some of the compressive load. The compressive forces (on the applied load side of the bone shaft) are preferably primarily transferred through shear forces at the interface between the bone shaft and the system 10. Tensile forces (on the opposing side) are resisted by the structural frame 20 and/or surgical fluid 40, and also transferred via shear forces at the bone-system interface so that the system 10 substantially bears or carries such forces.

In general, the elements of the system 10 cooperate to accomplish fracture fixation and stabilization to a greater degree than would any single component by itself. The combination of the sheath 30 partially enveloping the structural frame 20 which is embedded in a hardened column of surgical fluid 40 form a cooperative structure that offers fixation and stabilization that is superior to other methods that may use one or more similar elements. This represents an advance in the art through the synthesis of multiple components, installed using the technique or method described, and cooperating together to provide improved fixation and stabilization.

The system 10 may remain in place, without requiring later removal. In fact, the system 10 may be therapeutic in the later phases of fracture healing, including cellular proliferation, callous formation, bony union (ossification), and remodeling. In one embodiment, the system 10 of the present invention eventually performs a secondary role, after the fracture healing process progresses and the new bone achieves a shape and density capable of withstanding the forces of normal use.

Retrieval: Use of the system 10 may include removing the components after the healing phase. By way of example and not limitation, Figures 48-50 illustrate the retrieval and removal of certain components of the system 10. To accomplish retrieval and removal, an access opening 450 may be made through an end of the bone or, alternatively, such removal may be achieved through the breach 80 that was used for insertion of the system 10 initially. As shown in Figures 37 and 48, a retrieval tool 290 may be used to facilitate the manipulation of the structural frame 20 and related components. The retrieval tool 290 may comprise a proximal end and a generally opposing distal end. The proximal end may include a handle or may be otherwise graspable. The distal end may include a hook 292 disposed on the tip. The hook 292 may be used to engage the structural frame 20 or one or more other elements.

As shown in Figure 48, the distal end of the retrieval tool 290 may be inserted through the opening 450 or the breach 80 to engage the guide wire 156. The retrieval tool 290 may be turned, twisted, or otherwise manipulated to grasp the guide wire 156. As shown in Figure 49, the guide wire 156 may also be turned, twisted, manipulated, or otherwise altered into a shape that is readily graspable by the hook 292 of the retrieval tool 290. As shown in Figure 50, the structural frame 20 may be adapted to collapse when a tensile force (stretching) is applied, to assist in its removal. In one example, the structural frame 20 may be similar to the alternate structural framework 270 illustrated in Figures 24-27, in that it may be adapted such that movement of the guide wire 156 in a generally proximal direction causes the frame 20 to contract or otherwise collapse to its unexpanded position. The frame 20, the guide wire 145, and the restrictor 45 may be removed together through the opening 450.

### Conclusion

Although the systems herein have been illustrated by describing examples, and while the examples have been described in considerable detail, the description is not exhaustive. Accordingly, this application is intended to embrace alterations, modifications, and variations that fall within the scope of the appended claims. Furthermore, the preceding description is not meant to limit the scope of the invention.

## Claims

1. A systems (10) for treating a fracture site (110) in a bone (100) having an intramedullary canal (120), said system (10) comprising: a structural frame (20) having a size and shape suitable for insertion into said canal (120), having a length sufficient to span said fracture site (110), and having a contour adapted to engage an internal surface of said canal (120) ; said system (10) being **characterized in that** comprises a sheath (30) having a size and shape suitable for insertion into said canal (120), having a length sufficient to span said fracture site (110), and having an elongate tubular structure sized and shaped to receive said structural frame (20), said sheath (30) being made of a material defining a plurality of pores, said pore size is selected to be substantially impermeable to a surgical fluid (40), thereby reducing intrusion of said fluid (40) into said fracture site (110).

2. The system (10) of claim 1, further **characterized in that** said structural frame (20) is expandable from a first cross-sectional size to a second cross-sectional size which is larger than said first size.

3. The system (10) of claim 2, further **characterized in that** said structural frame (20) is biased toward expansion to said second size, such that said structural frame (20) requires a retainer in order to remain at said first size.

4. The system (10) of claim 2, further **characterized in that** said structural frame (20) is configured to receive an expandable member which, upon expansion, expands said structural frame (20) from said first size to said second size.

5. The system (10) of claim 2, further **characterized in that**, after expansion, said structural frame (20) is collapsible from said second size to said first size.

6. The system (10) of claim 2, further **characterized in that**, after expansion, said structural frame (20) is collapsible from said second size to said first size and retrievable from said canal (120).

7. The system (10) of claim 2, further **characterized in that** said structural frame (20) is biased toward remaining at said second size once expanded.

8. The system (10) of claim 2, further **characterized in that** said structural frame (20) resists collapsing forces once expanded to said second size.

9. The system (10) of claim 2, further, **characterized by** a locking member configured to maintain said structural frame (20) at said second size and resist collapsing forces.

10. The system (10) of claim 1, wherein the structural frame (20) is **characterized by** a generally hollow, substantially tubular shape.

11. The system (10) of claim 1, further **characterized in that** said structural frame (20) is made of a biocompatible material.

12. The system (10) of claim 1, further **characterized in that** said structural frame (20) is made of a bioabsorbable material.

13. The system (10) of claim 1, further **characterized in that** said structural frame (20) carries a therapeutic agent.

14. The system (10) of claim 1, wherein the structural frame (20) is further **characterized by** a plurality of discrete components nested together.

15. The system (10) of claim 1, wherein the structural frame (20) is further **characterized by** a plurality of elongate members connected to one another by one or more linking members.

16. The system (10) of claim 1, wherein the structural frame (20) is further **characterized by**: a generally central elongate shaft; and a plurality of other elongate members, each connected to said shaft by one or more linking members.

17. The system (10) of claim 1, wherein the structural frame (20) is further **characterized by**: a generally central elongate shaft sized and shaped to receive a guide wire (156) having near its distal end one or more fins (158) disposed thereon; a plurality of other elongate members, each connected to said shaft by one or more linking members, said one or more linking members positioned such that distal motion against said one or more fins (158) urges said plurality of other elongate members away from said shaft, thereby expanding said structural frame (20).

18. The system (10) of claim 1, wherein the structural frame (20) is further **characterized by** a plurality of prongs (24) configured to engage an internal surface of said canal (120).

19. The system (10) of claim 1, further **characterized in that** said structural frame (20) is sized and shaped to receive the sheath (30) along at least a portion of the length of said structural frame (20), said sheath (30) having a length sufficient to span said fracture site.

20. The system (10) of claim 1, further **characterized in that** the structural frame (20) has an elongate size and shape suitable for engagement with a prosthesis (500) in said canal (120) and has a length sufficient to span a periprosthetic fracture site (510) and engage said prosthesis (500).

21. The system (10) of claim 1, wherein the structural frame (20) is further **characterized by**: an extension member having a first end connected to said structural frame (20) and a generally opposing second end sized and shaped for engagement with a prosthesis (500) in said canal (120).

22. The system (10) of claim 1, further **characterized by** a strain gage connected to the structural frame (20) and positioned across said fracture site (110), a transmitter, and a receiver.

23. The system (10) of claim 1, wherein the structural frame (20) is further **characterized by** an electrical osteogenic stimulator positioned near said fracture site (110).

24. The system (10) of claim 1, wherein the sheath (30) is further **characterized in that** said elongate tubular structure is sized and shaped to allow a portion of said structural frame (20) to extend through and beyond each end of said sheath (30), said structural frame (20) having a contour adapted to engage an internal surface of said canal (120).

25. The system (10) of claim 1, wherein the sheath (30) is further **characterized in that** said elongate tubular structure is sized and shaped to be received within said structural frame (20).

26. The system (10) of claim 1, further **characterized in that** said sheath (30) is made of an elastic material.

27. The system (10) of claim 1, wherein the sheath (30) is further **characterized by** a plurality of layers.

28. The system (10) of claim 1, further **characterized in that** said sheath (30) is made of a biocompatible material.

29. The system (10) of claim 1, further **characterized in that** said sheath (30) is made of a osteoconductive material.

30. The system (10) of claim 1, further **characterized in that** said sheath (30) is made of a osteoinductive material.

31. The system (10) of claim 1, further **characterized in that** said sheath (30) is made of a bioabsorbable material.

32. The system (10) of claim 1, further **characterized in that** said sheath (30) carries a therapeutic agent.

33. The system (10) of claim 1, further **characterized in that** said sheath (30) carries a therapeutic agent disposed in an outer surface of said sheath (30).

34. The system (10) of claim 1, further **characterized in that** said sheath (30) carries a therapeutic agent disposed in a three-dimensional matrix throughout said sheath (30).

35. The system (10) of claim 1, further **characterized in that** said sheath (30) covers at least a portion of the length of said structural frame (20), such that said sheath (30) spans said fracture site (110).

36. The system (10) of claim 1, further **characterized in that** said sheath (30) may be placed within said structural frame (20) along at least a portion of the length of said structural frame (20), such that said sheath (30) spans said fracture site (110).

37. The system (10) of claim 1, further **characterized in that** said sheath (30) is made of an elastic material to accommodate expansion of said structural frame (20).

38. The system (10) of claim 1, further **characterized by** a generally hollow, substantially tubular shape.

39. The system (10) of claim 1, further **characterized in that** said system (10) carries a therapeutic agent.

40. The system (10) of claim 1, further **characterized by** a plurality of prongs (24) sized and shaped to engage an internal surface of said canal (120).

41. The system (10) of claim 1, further **characterized by** an electrical osteogenic stimulator positioned near said fracture site (110).

42. The system (10) of claim 1, further **characterized in that** said structural frame (20) comprises: a generally central elongate shaft sized and shaped to receive a guide wire (156) having near its distal end one or more fins (158) disposed thereon; a plurality of other elongate members, each connected to said shaft by one or more linking members, said one or more linking members positioned such that distal motion against said one or more fins (158) urges said plurality of other elongate members away from said shaft, thereby expanding said structural frame (20).

43. The system (10) of any one of claims 1 to 42 **characterized in that** it comprises a hardenable surgical fluid (40) cooperative with said structural frame (20) to provide additional support across said fracture site (110).

44. The system (10) of claim 43, further **characterized in that** said hardenable surgical fluid (40) substantially fills the space within said sheath (30) and substantially fills at least a portion of the length of said structural frame (20).

45. The system (10) of claim 43, further **characterized in that** said sheath (30) is made of a material defining a plurality of pores, said pore size selected to be substantially impermeable to said surgical fluid (40), thereby reducing intrusion of said fluid (40) into said fracture site (110).

46. The system (10) of claim 43, further **characterized in that** said sheath (30) is made of an elastic material to accommodate expansion of said structural frame (20).

47. The system (10) of claim 43, further **characterized in that** said structural frame (20 and sheath (30) together form a generally hollow, substantially tubular shape capable of receiving an injection of said hardenable surgical fluid (40).

48. The system (10) of claim 43, further **characterized in that** it comprises: a restrictor (45) placed at a site within said canal (120), sized and shaped to restrict a quantity of said hardenable surgical fluid (40) from extending beyond said site.

49. The system (10) of claim 43, further **characterized in that** said structural frame (20) and said hardenable surgical fluid (40) together resist collapsing forces once said frame (20) is expanded to said second size.

## Patentansprüche

1. System (10) für die Behandlung einer Frakturstelle (110) an einem Knochen (100) mit einem intramedullären Kanal (120), wobei das besagte System (10) umfasst: ein Stützgerüst (20) mit einer Größe und Form, die für das Einsetzen in den besagten Kanal (120) geeignet ist, mit einer Länge, die ausreicht, sich über die besagte Frakturstelle (110) zu erstrecken, und mit einer Kontur, die angepasst ist, um in eine innere Oberfläche des besagten Kanals (120) zu greifen; **dadurch gekennzeichnet, dass** das besagte System (10) eine Manschette (30) umfasst, mit einer Größe und Form, die für das Einsetzen in den besagten Kanal (120) geeignet ist, mit einer Länge, die ausreicht sich über die gesamte Frakturstelle (110) zu erstrecken, und mit einer länglichen rohrförmigen Struktur, deren Größe und Form zur Aufnahme des besagten Stützgerüsts (20) geeignet ist, wobei die besagte Manschette (30) aus einem Material besteht, das eine Vielzahl von Poren definiert, wobei die Größe der besagten Poren so ausgewählt ist, dass diese für ein Operationsfluid (40) im Wesentlichen impermeabel sind, wodurch das Eindringen des besagten Fluids (40) in die besagte Frakturstelle (110) vermindert wird.

2. System (10) nach Anspruch 1, **dadurch gekennzeichnet, dass** das besagte Stützgerüst (20) von einer ersten Querschnittsgröße auf eine zweite Querschnittsgröße, die größer ist als die erste Querschnittgröße, ausdehnbar ist.

3. System (10) nach Anspruch 2, **dadurch gekennzeichnet, dass** das besagte Stützgerüst (20) für die Ausdehnung auf die besagte zweite Größe vorgespannt ist, so dass das Stützgerüst (20) eine Feststelleinrichtung benötigt, damit es in der ersten Größe verbleibt.

4. System (10) nach Anspruch 2, **dadurch gekennzeichnet, dass** das besagte Stützgerüst (20) für die Aufnahme eines ausdehnbaren Elements eingerichtet ist, das bei Ausdehnung das besagte Stützgerüst (20) von der besagten ersten Größe auf die besagte zweite Größe ausdehnt.

5. System (10) nach Anspruch 2, **dadurch gekennzeichnet, dass** das besagte Stützgerüst (20) nach der Ausdehnung von der besagten zweiten Größe auf die besagte erste Größe reduzierbar ist.

6. System (10) nach Anspruch 2, **dadurch gekennzeichnet, dass** das besagte Stützgerüst (20) nach der Ausdehnung von der besagten zweiten Größe zur besagten ersten Größe reduzierbar ist und aus dem besagten Kanal (120) entfernbar ist.

7. System (10) nach Anspruch 2, **dadurch gekennzeichnet, dass** das besagte Stützgerüst (20) vorgespannt ist, um nach einer Ausdehnung in der besagten zweiten Größe zu verbleiben.

8. System (10) nach Anspruch 2, **dadurch gekennzeichnet, dass** das besagte Stützgerüst (20) nach einer Ausdehnung auf die besagte zweite Größe reduzierenden Kräften widersteht.

9. System (10) nach Anspruch 2, weiter **gekennzeichnet durch** eine Verriegelungskomponente, die das besagte Stützgerüst (20) in der zweiten besagten Größe hält und reduzierenden Kräften widersteht.

10. System (10) nach Anspruch 1, wobei das Stützgerüst (20) durch eine im Allgemeinen hohle, im Wesentlichen rohrförmige Form **gekennzeichnet** ist.

11. System (10) nach Anspruch 1, **dadurch gekennzeichnet, dass** das besagte Stützgerüst (20) aus einem biokompatiblen Material besteht.

12. System (10) nach Anspruch 1, **dadurch gekennzeichnet, dass** das besagte Stützgerüst (20) aus einem bioabsorbierbaren Material besteht.

13. System (10) nach Anspruch 1, **dadurch gekennzeichnet, dass** das besagte Stützgerüst (20) ein therapeutisches Mittel aufweist.

14. System (10) nach Anspruch 1, wobei das Stützgerüst (20) durch eine Vielzahl von Einzelkomponenten **gekennzeichnet** ist, die miteinander verbunden sind.

15. System (10) nach Anspruch 1, wobei das Stützgerüst (20) durch eine Vielzahl von länglichen Elementen **gekennzeichnet** ist, die durch ein oder mehrere Verbindungselemente miteinander verbunden sind.

16. System (10) nach Anspruch 1, wobei das Stützgerüst (20) durch einen im Allgemeinen mittigen, länglichen Schaft und eine Vielzahl von anderen länglichen Elementen **gekennzeichnet** ist, von denen jedes durch ein oder mehrere Verbindungselemente mit dem besagten Schaft verbunden ist.

17. System (10) nach Anspruch 1, bei dem das Stützgerüst (20) **gekennzeichnet ist durch** einen im Allgemeinen mittigen, länglichen Schaft, dessen Größe und Form für die Aufnahme eines Führungsdrahts (156) ausgelegt sind, an dem in der Nähe seines distalen Endes eine oder mehrere Finnen (158) angeordnet sind, sowie **durch** eine Vielzahl von anderen länglichen Elementen, von denen jedes **durch** ein oder mehrere Verbindungselemente mit dem besagten Schaft verbunden ist, wobei die besagten ein oder mehreren Verbindungselemente so positioniert sind, dass die Vielzahl von anderen länglichen Elementen **durch** eine distale Bewegung gegen die besagten eine oder mehreren Finnen (158) von dem besagten Schaft weg getrieben werden, und **dadurch** das besagte Stützgerüst (20) ausdehnen.

18. System (10) nach Anspruch 1, wobei das Stützgerüst (20) durch eine Vielzahl von Zacken (24) **gekennzeichnet** ist, die so ausgelegt sind, dass sie in die innere Oberfläche des besagten Kanals (120) greifen.

19. System (10) nach Anspruch 1, **dadurch gekennzeichnet, dass** das besagte Stützgerüst (20) über eine Größe und Form verfügt, die zur Aufnahme der Manschette (30) entlang mindestens eines Teils des besagten Stützgerüsts (20) geeignet ist, wobei die Manschette (30) eine Länge hat, die ausreicht, sich über die gesamte Frakturstelle zu erstrecken.

20. System (10) nach Anspruch 1, **dadurch gekennzeichnet, dass** das Stützgerüst (20) eine längliche Größe und Form aufweist, die für das Ineinandergreifen mit einer Prothese (500) im besagten Kanal (120) geeignet sind und eine Länge hat, die ausreicht, sich über die gesamte periprothetische Frakturstelle (510) zu erstrecken und mit der besagten Prothese (500) ineinanderzugreifen.

21. System (10) nach Anspruch 1, wobei das Stützgerüst (20) **gekennzeichnet ist durch** ein Verlängerungselement mit einem ersten Ende, das mit dem besagten Stützgerüst (20) verbunden ist, und einem im Allgemeinen entgegengesetzten zweiten Ende, das über eine Größe und Form verfügt, die für das Ineinandergreifen mit einer Prothese (500) im besagten Kanal (120) geeignet sind.

22. System (10) nach Anspruch 1, **gekennzeichnet durch** einen Dehnungsmesser, der mit dem Stützgerüst (20) verbunden ist und entlang der besagten Frakturstelle (110) positioniert ist, einen Sender, und einen Empfänger.

23. System (10) nach Anspruch 1, wobei das Stützgerüst (20) durch einen elektrischen osteogenen Stimulator **gekennzeichnet** ist, der in der Nähe der besagten Frakturstelle (110) positioniert ist.

24. System (10) nach Anspruch 1, wobei die Manschette (30) **dadurch gekennzeichnet ist, dass** die besagte längliche rohrförmige Struktur über eine Größe und Form verfügt, die es ermöglichen, dass ein Teil des besagten Stützgerüsts (20) sich durch und über beide Enden der besagten Manschette (30) hinaus erstreckt, wobei das besagte Stützgerüst (20) eine Kontur hat, die so angepasst ist, dass sie in eine innere Oberfläche des besagten Kanals (120) greift.

25. System (10) nach Anspruch 1, wobei die Manschette (30) **dadurch gekennzeichnet ist, dass** die besagte längliche rohrförmige Struktur über eine Größe und Form zur Aufnahme innerhalb des besagten Stützgerüsts (20) verfügt.

26. System (10) nach Anspruch 1, **dadurch gekennzeichnet, dass** die besagte Manschette (30) aus einem elastischen Material besteht.

27. System (10) nach Anspruch 1, wobei die Manschette (30) durch eine Vielzahl von Schichten **gekennzeichnet** ist.

28. System (10) nach Anspruch 1, **dadurch gekennzeichnet, dass** die besagte Manschette (30) aus einem biokompatiblen Material besteht.

29. System (10) nach Anspruch 1, **dadurch gekennzeichnet, dass** die besagte Manschette (30) aus einem osteokonduktiven Material besteht.

30. System (10) nach Anspruch 1, **dadurch gekennzeichnet, dass** die besagte Manschette (30) aus einem osteoinduktiven Material besteht.

31. System (10) nach Anspruch 1, **dadurch gekennzeichnet, dass** die besagte Manschette (30) aus einem bioabsorbierbaren Material besteht.

32. System (10) nach Anspruch 1, **dadurch gekennzeichnet, dass** die besagte Manschette (30) ein therapeutisches Mittel trägt.

33. System (10) nach Anspruch 1, **dadurch gekennzeichnet, dass** die besagte Manschette (30) ein therapeutisches Mittel trägt, das in einer äußeren Oberfläche der besagten Manschette (30) enthalten ist.

34. System (10) nach Anspruch 1, **dadurch gekennzeichnet, dass** die besagte Manschette (30) ein therapeutisches Mittel trägt, das in einer dreidimensionalen Matrix über die gesamte besagte Manschette (30) hinweg enthalten ist.

35. System (10) nach Anspruch 1, **dadurch gekennzeichnet, dass** die besagte Manschette (30) mindestens einen Teil des besagten Stützgerüsts (20) in Längsrichtung abdeckt, sodass sich die besagte Manschette (30) über die gesamte Frakturstelle (110) erstreckt.

36. System (10) nach Anspruch 1, **dadurch gekennzeichnet, dass** die besagte Manschette (30) entlang mindestens eines Teils des besagten Stützgerüsts (20) in Längsrichtung so in dem besagten Stützgerüst (20) platziert werden kann, dass sich die besagte Manschette (30) über die besagte Frakturstelle (110) erstreckt.

37. System (10) nach Anspruch 1, **dadurch gekennzeichnet, dass** die besagte Manschette (30) aus einem elastischen Material besteht, das eine Ausdehnung des besagten Stützgerüsts (20) zulässt.

38. System (10) nach Anspruch 1, weiter **gekennzeichnet durch** eine im Allgemeinen hohle, im Wesentlichen rohrförmige Form.

39. System (10) nach Anspruch 1, **dadurch gekennzeichnet, dass** das besagte System (10) ein therapeutisches Mittel trägt.

40. System (10) nach Anspruch 1, weiter **gekennzeichnet durch** eine Vielzahl von Zacken (24), die eine Größe und Form zum Eingriff in eine innere Oberfläche des besagten Kanals (120) aufweisen.

41. System (10) nach Anspruch 1, weiter **gekennzeichnet durch** einen elektrischen osteogenen Stimulator, der in der Nähe der besagten Frakturstelle (110) positioniert ist.

42. System (10) nach Anspruch 1, **dadurch gekennzeichnet, dass** das besagte Stützgerüst (20) Folgendes umfasst: einen im Allgemeinen mittigen, länglichen Schaft, dessen Größe und Form für die Aufnahme eines Führungsdrahts (156) ausgelegt sind, an dem in der Nähe seines distalen Endes eine oder mehrere Finnen (158) angeordnet sind, sowie eine Vielzahl von anderen länglichen Elementen, von denen jedes durch ein oder mehrere Verbindungselemente mit dem besagten Schaft verbunden ist, wobei die besagten ein oder mehreren Verbindungselemente so positioniert sind, dass die Vielzahl von anderen länglichen Elementen durch eine distale Bewegung gegen die besagten eine oder mehreren Finnen (158) von dem besagten Schaft weg getrieben werden, und **dadurch** das besagte Stützgerüst (20) ausdehnen.

43. System (10) nach einem der Ansprüche 1 bis 42, **dadurch gekennzeichnet, dass** es ein härtbares Operationsfluid (40) umfasst, das zusammen mit dem besagten Stützgerüst (20) zusätzlichen Halt an der besagten Frakturstelle (110) bietet.

44. System (10) nach Anspruch 43, **dadurch gekennzeichnet, dass** das besagte härtbare Operationsfluid (40) den Raum innerhalb der besagten Manschette (30) im Wesentlichen ausfüllt und mindestens einen Teil der Länge des besagten Stützgerüsts (20) im Wesentlichen ausfüllt.

45. System (10) nach Anspruch 43, **dadurch gekennzeichnet, dass** die besagte Manschette (30) aus einem Material besteht, das eine Vielzahl von Poren definiert, wobei die Größe der besagten Poren so ausgewählt ist, dass diese für ein Operationsfluid (40) im Wesentlichen impermeabel sind, wodurch das Eindringen des besagten Fluids (40) in die besagte Frakturstelle (110) vermindert wird.

46. System (10) nach Anspruch 43, **dadurch gekennzeichnet, dass** die besagte Manschette (30) aus einem elastischen Material besteht, das eine Ausdehnung des besagten Stützgerüsts (20) zulässst.

47. System (10) nach Anspruch 43, **dadurch gekennzeichnet, dass** das besagte Stützgerüst (20) und die besagte Manschette (30) zusammen eine im Allgemeinen hohle, im Wesentlichen rohrförmige Form bilden, die in der Lage ist, eine Injektion des besagten härtbaren Operationsfluids (40) aufzunehmen.

48. System (10) nach Anspruch 43, **dadurch gekennzeichnet, dass** es Folgendes umfasst: ein Drosselventil (45), das an einer Stelle im besagten Kanal (120) platziert wird, mit einer solchen Größe und Form, dass die Ausdehnung einer Menge des besagten härtbaren Operationsfluids (40) über die besagte Stelle hinweg beschränkt wird.

49. System (10) nach Anspruch 43, **dadurch gekennzeichnet, dass** das besagte Stützgerüst (20) und das besagte härtbare Operationsfluid (40) nach einer Ausdehnung des besagten Gerüsts (20) auf die besagte zweite Größe zusammen den reduzierenden Kräften widerstehen.

## Revendications

1. Dispositif (10) destiné à traiter un site de fracture (110) dans un os (100) ayant un canal intramédullaire (120), ledit dispositif (10) comprend une ossature structurelle (20) ayant une dimension et une forme appropriées pour l'insertion dans ledit canal (120), ayant une longueur suffisante pour couvrir ledit site de fracture (110) et ayant un profil adapté pour s'appuyer contre une surface interne dudit canal (120) ; ledit dispositif (10) étant **caractérisé en ce qu'**il comprend une gaine (30) ayant une dimension et une forme appropriées pour l'insertion dans ledit canal (120), ayant une longueur suffisante pour couvrir ledit site de fracture (110) et ayant une structure tubulaire allongée dimensionnée et conformée pour recevoir ladite ossature structurelle (20), ladite gaine (30) étant faite d'une matière définissant une pluralité de pores, la dimension desdits pores étant choisie de façon à être sensiblement imperméable à un fluide chirurgical (40), de sorte à réduire l'intrusion dudit fluide (40) dans ledit site de fracture (110).

2. Système (10 selon la revendication 1, **caractérisé en outre en ce que** ladite ossature structurelle (20) peut être dilatée d'une première dimension de section transversale à une deuxième dimension de section transversale qui est plus grande que ladite première dimension.

3. Système (10) selon la revendication 2, **caractérisé en outre en ce que** ladite ossature structurelle (20) est sollicitée dans le sens de la dilatation à ladite deuxième dimension, de sorte que ladite ossature structurelle (20) exige un élément de retenue pour rester à ladite première dimension.

4. Système (10) selon la revendication 2, **caractérisé en outre en ce que** ladite ossature structurelle (20) est configurée pour recevoir un élément dilatable qui, lors de sa dilatation, dilate de ladite ossature structurelle (20) de ladite première dimension à ladite deuxième dimension.

5. Système (10) selon la revendication 2, **caractérisé en outre en ce qu'**après la dilatation, ladite ossature structurelle (20) peut être rétractée de ladite deuxième dimension à ladite première dimension.

6. Système (10) selon la revendication 2, **caractérisé en outre en ce qu'**après la dilatation, ladite ossature structurelle (20) peut être rétractée de ladite deuxième dimension à ladite première dimension et être extraite dudit canal (120).

7. Système (10) selon la revendication 2, **caractérisé en outre en ce que** ladite ossature structurelle (20) est sollicitée vers le maintien à ladite deuxième dimension lorsqu'elle a été dilatée.

8. Système (10) selon la revendication 2, **caractérisé en outre en ce que** ladite ossature structurelle (20) résiste aux forces de retrait lorsqu'elle a été dilatée à ladite deuxième dimension.

9. Système (10) selon la revendication 2, **caractérisé en outre par** un élément de verrouillage configuré pour maintenir ladite ossature structurelle (20) à ladite deuxième dimension et pour résister aux forces de retrait.

10. Système (10) selon la revendication 1, dans lequel l'ossature structurelle (20) est **caractérisée par** une conformation générale creuse, sensiblement tubulaire.

11. Système (10) selon la revendication 1, **caractérisé en outre en ce que** ladite ossature structurelle (20) est faite d'une matière biocompatible.

12. Système (10) selon la revendication 1, **caractérisé en outre en ce que** ladite ossature structurelle (20) est faite d'une matière bioabsorbable.

13. Système (10) selon la revendication 1, **caractérisé en outre en ce que** ladite ossature structurelle (20) porte un agent thérapeutique.

14. Système (10) selon la revendication 1, dans lequel l'ossature structurelle (20) est **caractérisée en outre par** une pluralité de composants distincts emboîtés les uns dans les autres.

15. Système (10) selon la revendication 1, dans lequel l'ossature structurelle (20) est **caractérisée en outre par** une pluralité d'éléments allongés reliés l'un à l'autre par un ou plusieurs éléments de liaison.

16. Système (10) selon la revendication 1, dans lequel l'ossature structurelle (20) est **caractérisée en outre par** : une tige allongée, de façon générale centrale ; et une pluralité d'autres éléments allongés, dont chacun est relié à ladite tige par un ou plusieurs éléments de liaison.

17. Système (10) selon la revendication 1, dans lequel l'ossature structurelle (20) **caractérisée en outre par** une tige allongée, de façon générale centrale, dimensionnée et configurée pour recevoir un fil guide (156) ayant une ou plusieurs ailettes (158) disposées sur lui près de son extrémité distale ; une pluralité d'autres éléments allongés, dont chacun est relié à ladite tige par un ou plusieurs éléments de liaison, lesdits un ou plusieurs éléments de liaison étant positionnés de telle manière qu'un mouvement distal qui les applique contre lesdites une ou plusieurs ailettes (158) tende à écarter ladite pluralité d'autres éléments allongés de ladite tige, en dilatant ainsi ladite ossature structurelle (20).

18. Système (10) selon la revendication 1, dans lequel l'ossature structurelle (20) est **caractérisée en outre par** une pluralité de pointes (24) configurées pour attaquer une surface interne dudit canal (120).

19. Système (10) selon la revendication 1, **caractérisé en outre en ce que** ladite ossature structurelle (20) est dimensionnée et configurée pour recevoir la gaine (30) le long d'au moins une partie de la longueur de ladite ossature structurelle (20), ladite gaine (30) ayant une longueur suffisante pour couvrir ledit site de fracture.

20. Système (10) selon la revendication 1, **caractérisé en ce que** l'ossature structurelle (20) a une dimension et une forme allongées appropriées pour coopérer avec une prothèse (500) située dans ledit canal (120) et a une longueur suffisante pour couvrir un site de fracture périprosthétique (510) et pour coopérer avec ladite prothèse (500).

21. Système (10) selon la revendication 1, dans lequel l'ossature structurelle (20) est **caractérisée en outre par** un élément prolongateur ayant une première extrémité reliée à ladite ossature structurelle (20) et une deuxième extrémité sensiblement opposée et dimensionnée et configurée pour coopérer avec une prothèse (500) située dans ledit canal (120).

22. Système (10) selon la revendication 1, **caractérisé par** une jauge de contrainte reliée à l'ossature structurelle (20) et positionnée à travers ledit site de fracture (110), un émetteur et un récepteur.

23. Système (10) selon la revendication 1, dans lequel l'ossature structurelle (20) est **caractérisée en outre par** un stimulateur ostéogénique électrique positionné à proximité dudit site de fracture (110).

24. Système (10) selon la revendication 1, dans lequel la gaine (30) est **caractérisée en outre en ce que** ladite structure tubulaire allongée est dimensionnée et configurée pour permettre à une partie de ladite ossature structurelle (20) de s'étendre sur toute la longueur, et au-delà de chacune des extrémités, de ladite gaine (30), ladite ossature structurelle (20) ayant un profil adapté pour s'appuyer contre une surface interne dudit canal (120).

25. Système (10) selon la revendication 1, dans lequel la gaine (30) est **caractérisée en outre en ce que** ladite structure tubulaire allongée est dimensionnée et configurée pour être reçue à l'intérieur de ladite ossature structurelle (20).

26. Système (10) selon la revendication 1, **caractérisé en outre en ce que** ladite gaine (30) est faite d'une matière élastique.

27. Système (10) selon la revendication 1. dans laquelle la gaine (30) est **caractérisée en outre par** une pluralité de couches.

28. Système (10) selon la revendication 1, **caractérisé en outre en ce que** ladite gaine (30) est faite d'une matière biocompatible.

29. Système (10) sur la revendication 1, **caractérisé en ce que** ladite gaine (30) est faite d'une matière ostéoconductrice.

30. Système (10) selon la revendication 1, **caractérisé en outre en ce que** ladite gaine (30) est faite d'une matière ostéoinductrice.

31. Système (10) selon la revendication 1, **caractérisé en outre en ce que** ladite gaine (30) est faite d'une matière bioabsorbable.

32. Système (10) selon la revendication 1, **caractérisé en outre en ce que** ladite gaine (30) porte un agent thérapeutique.

33. Système (10) selon la revendication 1, **caractérisé en outre en ce que** ladite gaine (30) porte un agent thérapeutique disposé dans une surface extérieure de ladite gaine (30).

34. Système (10) selon la revendication 1, **caractérisé en outre en ce que** ladite gaine (30) porte un agent thérapeutique disposé dans une matrice tridimensionnelle dans toute l'étendue de ladite gaine (30).

35. Système (10) selon la revendication 1, **caractérisé en outre en ce que** ladite gaine (30) couvre au moins une partie de la longueur de ladite ossature structurelle (20) de telle manière que ladite gaine (30) couvre ledit site de fracture (110).

36. Système (10) selon la revendication 1, **caractérisé en outre en ce que** ladite gaine (30) peut être placée dans ladite ossature structurelle (20) le long d'au moins une partie de la longueur de ladite ossature structurelle (20), de telle manière que ladite gaine (30) couvre ledit site de fracture (110).

37. Système (10) selon la revendication 1, **caractérisé en outre en ce que** ladite gaine (30) est faite d'une matière élastique afin de s'adapter à la dilatation de ladite ossature structurelle (20).

38. Système (10) selon la revendication 1, **caractérisé en outre par** une conformation de forme générale creuse, sensiblement tubulaire.

39. Système (10) selon la revendication 1, **caractérisé en outre en ce que** ledit système (10) porte un agent thérapeutique.

40. Système (10) selon la revendication 1, **caractérisé en outre par** une pluralité de pointes (24) dimensionnées et configurées pour attaquer une surface interne dudit canal (120).

41. Système (10) selon la revendication 1, **caractérisé en outre par** un stimulateur ostéogénique positionné à proximité du site de fracture (110).

42. Système (10) selon la revendication 1, **caractérisé en outre en ce que** ladite ossature structurelle (20) comprend une tige allongés de façon générale centrale, dimensionnée et configurée pour recevoir un fil guide (156) ayant près de son extrémité distale une ou plusieurs ailettes (158) disposées sur lui ; une pluralité d'autres éléments allongés, dont chacun est relié à ladite tige par un ou plusieurs éléments de liaison, lesdits un ou plusieurs éléments de liaison étant positionnés de telle manière qu'un déplacement distal qui les applique contre lesdites une ou plusieurs des ailettes (158) tende à écarter ladite pluralité d'autres éléments allongés de ladite tige, en dilatant ainsi ladite ossature structurelle (20).

43. Système (10) selon une quelconque des revendications 1 à 42, **caractérisé en ce qu'**il comprend un fluide chirurgical durcissable (40) qui peut coopérer avec ladite ossature structurelle (20) pour former un support additionnel à travers ledit site de fracture (110).

44. Système (10) selon la revendication 43, **caractérisé en ce que** ledit fluide chirurgicale durcissable (40) remplit sensiblement l'espace compris à l'intérieur de ladite gaine (30) et remplit sensiblement au moins une partie de la longueur de ladite ossature structurelle (20).

45. Système (10) selon la revendication 43, **caractérisé en outre en ce que** ladite gaine (30) est faite d'une matière qui définit une pluralité de pores, la dimension desdits pores étant choisie pour être sensiblement imperméable audit fluide chirurgical (40), en réduisant ainsi l'intrusion dudit fluide (40) dans ledit site de fracture (110).

46. Système (10) selon la revendication 43, **caractérisé en outre en ce que** ladite gaine (30) est faite d'une matière élastique pour s'adapter à la dilation de ladite ossature structurelle (20).

47. Système (10) selon la revendication 43, **caractérisé en outre en ce que** ladite ossature structurelle (20) et ladite gaine (30) forment ensemble une configuration de façon générale creuse, sensiblement tubulaire, capable de recevoir une injection dudit fluide chirurgical durcissable (40).

48. Système (10) selon la revendication 43, **caractérisé en outre en ce qu'**il comprend un limiteur (45) placé à un site à l'intérieur dudit canal (120), dimensionné et configuré de manière à limiter la quantité dudit fluide chirurgical durcissable (40) qui peut s'écouler au-delà dudit site.

49. Système (10) selon la revendication 43, **caractérisé en outre en ce qu'**ensemble, ladite ossature structurelle (20) et ledit fluide chirurgical durcissable (40) résistent à des forces de retrait lorsque ladite ossature (20) a été dilatée à ladite deuxième dimension.
